# EUROPEAN PATENT APPLICATION

(11) **EP 4 826 639 A1**
(43) Date of publication of application: **21.10.2026**
(21) Application number: 25171426.7
(22) Date of filing: 17.04.2025
(51) Int. Cl.: G06T 3/153, G06T 7/33, G06V 10/75

(54) **TECHNIQUE FOR DEFORMABLE REGISTRATION OF MEDICAL IMAGES**

(71) Applicant: Siemens Healthineers AG, 91301 Forchheim (DE)
(72) Inventor: Reaungamornrat, Sureerat, Havertown, PA, 19083 (US); Wohlfahrt, Patrick, 91054 Erlangen, (DE)
(74) Representative: Schwarz, Claudia

(57) **Abstract**

The invention relates to a technique for providing a deformable registration result of a second medical image to a first medical image. A first medical image comprising an anatomical structure is received. A second medical image comprising the anatomical structure is received. Nested iterations are performed, which comprise an iteration over levels of spatial resolution with increasing spatial resolution, and, at each level of spatial resolution, an iteration regarding a set of deformation parameters of an analytic deformable registration function for registering a deformation of the second medical image to the first medical image. The nested iterations terminate at a predetermined level of fines spatial resolution. A deformable registration result of registering the second medical image to the first medical image is provided, comprises utilizing the, after the terminating optimized, set of deformation parameters in the deformable registration function.

## Description

The present invention relates to a technique for providing a deformable registration result of a second medical image to a first medical image, in particular comprising a method, a computing device, a computer program product, and a computer-readable storage medium.

Deformable registration is one of the key enabling technologies of image-guided intervention, treatment monitoring, and longitudinal studies. However, conventional approaches do not yield accurate and reliable outcomes, hindering their application in real clinical workflows. Although non-learning iterative approaches offer more reliable and accurate registration outcomes than learning approaches for images exhibiting high image quality, their lengthy computation time is conventionally prohibitive. On the other hand, although learning approaches exhibit more robustness against poor image quality, they often lack the ability to resolve large motion and to model highly varying deformation. Additionally, due to small training sets, these learning approaches fail to resolve motions in unseen test data whose distributions are different from training data distributions. One of the main reasons for the inability to resolve large varying motion is the insufficient number of model parameters per output dimension (e.g., the dimension of non-parametric transformation, i.e., displacement field, is three times the number of image voxels) resulting in insufficient model capacity to learn the large distribution of possible local motions. However, the number of model parameters is limited by the available GPU memory and the input image size (i.e., image resolution). For example, one can train a learning model on small image volumes (allowing for a larger number of model parameters per output dimension) but loses the ability to resolve motion of detailed structures (due to severe downsample of image volumes).

Various learning and non-learning approaches for deformable registration have been proposed. The important appealing characteristics of learning-based approaches is their insensitivity to a certain extent of image degradation and the computational speed since they only involve a single forward pass (single iteration), compared to non-learning approaches. However, their inferior registration performance on unseen data and their inability to operate on native image resolutions prohibit their application in clinics.

To bypass the issue of the insufficient number of parameters per output dimension in deep learning (DL) approaches for deformable registration, recent work focuses on the test-time iterative optimization of neural fields [1]. Since they are an iterative approach, runtime tends to be longer than DL approaches (involving only a single forward pass/iteration). Although one can train a neural field with a single coordinate point for other applications, for deformable registration, imposition of penalties (e.g., smoothness, rigid motion of bone, incompressibility, etc.) require the knowledge of deformation in a local neighborhood. Thus, the GPU/CPU memory still limits the size of neural networks and input image volumes. However, since the network only needs to solve motion for a single image pair (compared to DL approaches trained on multiple pairs), neural field approaches require a smaller number of parameters per output dimension than DL approaches. Because of this and because neural field approaches optimize motion on each individual pair of test data (vs. DL trained on training data, not test data), neural fields demonstrate improved performance compared to DL approaches [1]. Nevertheless, both DL and neural field approaches suffer from the memory limitation, prohibiting their applications in resolving motion of detailed structures (such as lymph nodes, seminal vesicles, larynx, glottis, cochlea, optic nerve).

Conventional approaches [2-6] lack the ability to resolve large motion and sharp change of adjacent motion (in terms of magnitude and/or direction). They are also unable to model motion based on tissue properties (like all neural field and learning methods), i.e., causing deformation of bone and smooth coherence motion of an otherwise discontinuous sliding interface.

It is therefore an object of the present invention to provide a solution for performing a registration of one medical image to another medical image that is efficient in terms of computing resources, such as memory, and that improves at in terms of computational speed. Alternatively or in addition, it is an object to provide a solution for improving anatomical consistency and/or accuracy of the registration of one medical image to another medical image, which is capable of modelling large motion and/or large deformations, sharp changes in adjacent motion and/or deformation, and/or motion and/or deformation based on tissue properties.

This object is solved by a method for providing a deformable registration result of a second medical image to a first medical image, by a computing device, by a computer program (and/or computer program product), and by a computer-readable storage medium according to the appended independent claims. Advantageous aspects, features and embodiments are described in the dependent claims and in the following description together with advantages.

In the following, the invention will be described with respect to the claimed method first. Features, advantages or alternative embodiments, mentioned with respect to the method can be assigned to the other claimed objects (e.g. the computer program or a device, in particular the computing device, or system or a computer program product), and vice versa. In other words, a system, apparatus or device can be improved with features described or claimed in the context of the method, and vice versa. In this case, the functional features of the method are embodied by structural units of the apparatus or device or system and vice versa, respectively. The method may refer to a software implementation, and the device may refer to a hardware implementation (e.g. with a spatial physical structure) or a virtualization thereof. Generally, in computer science a software implementation and a corresponding hardware implementation (e.g. as an embedded system) are equivalent. Thus, for example, a method step for "storing" data may be performed with a storage unit and respective instructions to write data into the storage. For the sake of avoiding redundancy, although the device may also be used in the alternative embodiments described with reference to the method, these embodiments are not explicitly described again for the device. In principle, the respective device or apparatus claim is configured to carry out the claimed method.

As to a method aspect, a (in particular computer-implemented) method for providing a deformable registration result of a second medical image to a first medical image is provided. The method comprises a step of receiving a first medical image, the first medical image comprising at least one anatomical structure. The method further comprises a step of receiving a second medical image. The second medical image comprises the (in particular same or analogous) at least one anatomical structure. The method further comprises a step of performing nested iterations. The nested iterations comprise an iteration over levels of spatial resolution with increasing spatial resolution. The nested iterations further comprise, at each level of spatial resolution, an iteration regarding a set of deformation parameters of an analytic deformable registration function (briefly also: transfer function, deformation function, and/or deformation) for registering a deformation of the received second medical image to the received first medical image.

The nested iterations may comprise, for a predetermined level of spatial resolution, a step of determining an image dissimilarity between the received first medical image and the received second medical image. The image dissimilarity may be determined per unit volume (or unit area) related to the predetermined level of spatial resolution. The nested iterations may further comprise, for the predetermined level of spatial resolution, a step of selecting a plurality of active control points. The active control points may be located in regions of the first medical image and/or of the second medical image, in which the determined image dissimilarity exceeds (or at least equals to) a (in particular level-specific) predefined image dissimilarity threshold value. The nested iterations may further comprise, for the predetermined level of spatial resolution, a step of iteratively optimizing the set of deformation parameters by iteratively optimizing an objective function. The objective function may be a differentiable function of the analytic deformable registration function. The analytic deformable registration function may comprise a sum over analytic functions centered at the selected active control points.

The iteration over levels of spatial resolution may comprise proceeding to a further predetermined level of spatial resolution, which has a finer level of spatial resolution and/or a smaller unit volume (or unit area) than the previous predetermined level of spatial resolution, and repeating the steps of determining an image dissimilarity, selecting a plurality of active control points, and iteratively optimizing the set of deformation parameters at the further predetermined level of spatial resolution. The iteration over levels of spatial resolution may further comprise terminating the nested iterations if or when, for a predetermined level of spatial resolution with finest spatial resolution and/or with a predetermined smallest size of unit volume (or unit area), the set of deformation parameters is iteratively optimized (and/or has reached its optimum according to the nested iterations).

The method further comprises a step of providing a deformable registration result of registering the second medical image to the first medical image. The deformable registration result comprises utilizing the - after the optimization terminated - set of deformation parameters in the deformable registration function.

By the inventive technique, deformably registering one (e.g., the second) medical image to another (e.g., the first) medical image can be improved in terms of accuracy and speed. Alternatively or in addition, computing resources (in particular processing resources and memory resources) can be saved, allowing to perform the deformable registration in a short computational time (in particular in real-time) and/or on any computing device (e.g., a workstation in a medical facility, such as in an operating room and/or in a doctor's office), in particular with limited working memory space. Thereby, support for treatment (e.g., surgery, and/or radiotherapy) planning, for optical guidance during the treatment (e.g., surgery, and/or radiotherapy), and/or for post-treatment (e.g., surgery, and/or radiotherapy) controls, and/or treatment monitoring can be improved. Alternatively or in addition, a comparison of the two medical images for longitudinal studies, and/or for transversal studies, can be improved.

The deformable registration by means of the inventive technique can be particularly accurate and/or reliable. Alternatively or in addition, an optimization stability can be improved. Alternatively or in addition, a dependence of the registration quality on the image quality can be reduced, allowing in particular for previously unseen data to have a high level of resolution of the deformation (and/or motion) of detailed structures (e.g., of vessels as compared to organs, such as liver or kidney).

By iteratively optimizing the deformation parameters (also: transformation parameters) defined based on a selected plurality of active control points, the optimization problem can be reduced to computations relating to a number of relevant points. Alternatively or in addition, by using the selected plurality of active control points, the iterative optimizing of the deformation parameters can be parallelized and/or performed for each (or at least a subset of the plurality of) active control point(s) independently. The parallelizing of the optimizing can in particular be combined with a shared memory, thereby avoiding duplications of storing values of deformation parameters.

Alternatively or in addition, by iteratively optimizing the deformation parameters based on derivatives of an analytical function, problems of small amounts of training datasets can be avoided, which typically arise when using machine learning (ML) and/or any other trainable method for performing deformable registration.

The objective function may be a combination of image similarity metrics (or image dissimilarity metrics), feature similarity metrics (e.g., point similarity and/or mesh similarity), and/or penalty (and/or regularization) functions promoting physically-meaningful structural alignment between the first medical image and the deformation of the second medical image (also: the two input images). The penalty (and/or regularization) functions may typically be functions of derivatives of the analytic deformable registration function with respect to spatial positions. The iterative optimization may involve the update of the deformation parameters, such as by the derivative of the objective function with respect to the deformation parameters.

According to the inventive technique, the objective function is not only computed at (e.g., active and/or inactive) control points. For example, an image dissimilarity metric may be computed "everywhere" (e.g., at all voxels or pixels, or at a predetermined subset of the voxels or pixels) on the first medical image and on the deformation of the second medical image.

Alternatively or in addition, an objective function may be a combination of an image (dis)similarity metric, a point similarity metric, and penalty and/or regularization functions. Thus, the objective function is not necessarily a function of derivatives of the deformation with respect to deformation parameters.

The inventive technique allows to perform deformable registration in the presence of highly varying deformation, large "motions", and/or sharp changes of adjacent deformations (and/or motions) in terms of magnitude and/or direction.

A longitudinal study may refer to tracking a patient's health state (e.g., in relation to the at least one anatomical structure) over time. In this case, the first medical image and the second medical image may be obtained from the same patient (e.g., a human or an animal, in particular a mammal, such as a dog or horse). Alternatively or in addition, a transversal study may refer to comparing the health states (e.g., in relation to the at least one anatomical structure) of different patients. In this case, the first medical image and the second medical image may be acquired from different patients.

The at least one anatomical structure comprised in the first medical image and the second medical image may comprise at least one identical (and/or analogous, and/or similar) anatomical structure, which may be comprised in a set of anatomical structures. E.g., the first medical image and the second medical image may only partly overlap, such as one (e.g., the first) medical image comprising an upper part of a patient's thorax and the other (e.g., the second) medical image comprising a lower part of the patient's thorax, with some overlap of the imaged anatomical structures in the center of the patient's thorax.

The at least one anatomical structure comprised in the first medical image and in the second medical image may in particular be analogous, and/or similar for different patients in terms of variations in size and/or shape across a population (or at least across a set of comparison candidates, such as patients of similar age, development state, and/or gender, e.g., grown-up men in a transversal study). Alternatively or in addition, the at least one anatomical structure comprised in the first medical image and in the second medical image may in particular be analogous, and/or similar for the same patient (and/or in a longitudinal study) in the sense of allowing for modifications due to surgery (e.g., removal of the patient's appendix or part of an organ, such as the lung) and/or due to a lesion (e.g., growth of a tumor and/or shrinking of a tumor upon treatment). E.g., both the first medical image and the second medical image may provide an image of a patient's organ (e.g., liver, kidney, lungs, and/or heart), bone structure, and/or (e.g., blood) vessels.

Examples of anatomical structures include, but are not limited to, organs, bone structures, and/or vessels.

The first medical image (briefly also: the first image) may serve as reference image for defining the deformable registration of the second medical image (briefly also: the second image) in order to map the imaged at least one (e.g., common, analogous, or similar) anatomical structure from the second image onto the first image. The first image is alternatively or in addition also denoted as still image. Alternatively or in addition, the second image is also denoted as deformation image, or as moving image.

By the deformable registration function, the at least one (e.g., common, analogous, or similar) anatomical structure, and/or organs comprised in both images, is/are deformed in the second image to match (or map to) the location in the first image.

The deformation may comprise the areas (and/or regions), where the deformable registration function acts non-trivially on the second medical image (e.g., not only as a global rotation of the second medical image). Acting non-trivially may comprise that the deformable registration function takes different values at different points in a neighbourhood.

Examples of functions relevant for deformable registration are radially symmetric functions (also: radial basis functions) such as a bump function, a Gaussian function, a Wendland function, a Wu function, a Buhmann function, and/or an oscillatory function (also: Gneiting function).

One way to view the deformation between the second and first image is as a motion, such as due to breathing for soft tissue, and/or a (in particular rigid) rotation, such as for mapping bone structures of the two images onto each other. E.g., the first image (e.g., a CT scan) may be acquired when the patient inhales, the lung is fully expanded, and/or the liver sits low within the thorax. Alternatively or in addition, the second image may be taken when the patient exhales, and/or the liver sits higher within the thorax. Such an image may, e.g., be taken during radiation therapy.

In the context of the invention, the expressions "motion" and "deformation" may essentially be used synonymously. Alternatively or in addition, a "moving image" may correspond to an image (such as the second medical image) that is deformed in order to register it to another image (such as the first medical image and/or a reference image).

In some cases, prior information with respect to structural deformations (e.g., induced by motion) and/or physiology are available, for example imaging results acquired for diagnostic purpose (e.g., acquisition of the first image) may be considered as pre-planning information for the second image. The prior information may comprise prior therapy information (e.g., an operating area, and/or applied radiation dose), a dose of a contrast agent, a type of a contrast agent, and/or a motion trajectory.

A deformation may cover, e.g., a range corresponding up to about 15cm in real life. E.g., the deformation may cover a range affected by the change in the lung volume between a fully inhaled and a fully exhaled state.

The deformation (and/or the motion) may be subject to one or more physical constraints (also: penalty terms and/or regularisation terms), such as rigidity of bone structures and/or limitations on deformations of soft tissue, such as relative motions of adjacent organs or limitations in a change of volume of an organ.

Using the physical constraints in relation to a tissue type may be based on a (e.g., semantic) segmentation. E.g., the first image and the second image may each (and/or independently) be segmented and/or comprise segmentation masks (also: segmentation labels).

Each medical image may be acquired by means of a medical imaging modality, and/or by means of a medical scanner. The scanner may be configured to provide a 3D medical image (also: volumetric image), which may correspond to a stack of slices of a 3D medical image, e.g., a CT scan and/or an MRT scan.

Each (e.g., the first and/or second) medical image may have been acquired independently by means of a 3D medical imaging modality. The 3D medical imaging modality may be (or may comprise) computed tomography (CT), magnetic resonance tomography (MRT), positron emission tomography (PET), single-photon emission computed tomography (SPECT), magnetic particle imaging (MPI), ultrasound (including, e.g., echocardiography, elastography, and/or photoacoustic imaging), and/or 3D heat maps.

The deformable registration may register a second 3D medical image to a first 3D medical image. Alternatively or in addition, it is possible to register a slice of a second 3D medical image to a first 2D medical image using the inventive technique. The motions may be assumed to be essentially 2D and/or map the slice (also: 2D slice) of the second 3D medical image to the first 2D medical image. Alternatively or in addition, the organ motions of the second 3D medical image may (in particular still) be 3D motions, and/or the slice may be selected for optimal mapping.

The 2D medical imaging modality may comprise radiography (also: X-ray imaging) and/or scintigraphy.

The inventive technique can be used for various medical imaging modalities. Alternatively or in addition, the inventive technique incorporates a computationally efficient algorithm for physically-constrained multimodality deformable registration. A parameterization of terms, such as contributions to the objective function, may depend on the medical imaging modality (or the pair of medical imaging modalities if the first medical image and the second medical image are acquired by means of different medical imaging modalities).

In some embodiments, the first image and the second image are acquired by means of the same (e.g., type of) medical imaging modality, and/or by means of the same scanner. E.g., two CT scans may be registered to each other, which may be acquired by means of the same scanner or by means of different scanners (e.g., having different technical specifications and/or different technical parameters). In further embodiments, the second image is acquired by means of a different medical imaging modality from the first image. E.g., the first image may be a CT scan, and the second image may correspond to an MRT scan.

The deformable registration function may transform a set of input points (in particular of one medical image) to a set of output points (in particular in a domain of another medical image). E.g., a set of input points of the second medical image may be transformed to a set of output points in the domain of the first medical image, in particular by applying the deformable registration function. Alternatively or in addition, a set of input points of the first medical image may be transformed to a set of output points in a domain of the second medical image, in particular by applying the inverse of the deformable registration function.

The deformable registration function may be an analytic function of the set of deformation parameters. An analytic function is generally a function that is locally given by a convergent power series.

The deformable registration function may in particular comprise a linear term comprising an affine matrix (and/or affine deformation parameters, and/or affine transformation parameters), an offset term and a sum of basis functions times warp coefficients,

An affine deformation is generally a deformation that can be completely described by an affine transformation. Such a transformation is generally composed of a linear transformation (such as rotation, shear, extension and/or compression) and a rigid body translation. Affine deformations may also be called homogeneous deformations.

The basis functions may have local support around a point, in particular around an (in particular initially active) control point and/or around landmarks comprised in the (e.g., first and/or second) image, and/or may be radial basis functions (RBFs).

Alternatively or in addition, the basis functions may have a tensor structure with size determined by a number of input points times the number of (in particular active) control points.

The (in particular radial) basis function may be parameterized by a center, which may be denoted as control point (cᵢ) in the context of the inventive technique, and a scalar value (s_{α}) defining a width of support (briefly also: support and/or width). A compactly-supported RBF (CSRBF) may have a bell-curve-like shape, and/or a bump-like shape. Alternatively or in addition, the parameters required to define the CSRBF may be similar to the parameters required to define a Gaussian function (in particular comprising center, cᵢ, and width, s_{α}). The scalar value (s_{α}) may, e.g., define a width of the CSRBF.

A control point being active may denote, in the context of the inventive technique, that the multiplicative prefactor, which may be denoted as warp coefficient, of the radial basis function centered there is in the process of being optimized.

A control point becoming inactive may denote, in the context of the inventive technique, that the optimization process for the multiplicative prefactor, and/or the warp coefficient, of the radial basis function centered there has been optimized, and/or has reached a constant value. Alternatively or in addition, after the control point has become inactive, the corresponding warp coefficient may be taken as fixed value (and/or frozen parameter) within the deformable registration function.

As the iteration proceeds to increasingly finer levels of spatial resolution, initial (and/or earlier) active control points may become inactive. In some embodiments, new active control points may appear, which may be associated with finer (and/or smaller) requirements of the deformation.

In some examples, a newly activated control point may coincide in its location with a by now inactive control point. The newly activated control point and the by now inactive control point may be the centers of different basis functions. E.g., a width may be different, and/or a warp coefficient may be different. In particular, to account for finer deformation structures, the newly activated control point may contribute with a smaller value. In case the maximal value of the basis function is normalized to a fixed value (e.g., to one), a finer deformation may correspond to a smaller value of the warp coefficient.

A landmark may be a point (and/or region) that is recognizable in each (e.g., first and/or second) image. Alternatively or in addition, a landmark may be a point, which upon deformation needs to be mapped to the same point in the second image and in the first image.

A radial basis function (RBF) is generally a real-valued function φ, whose value depends only on the distance between the input and some fixed point, either the origin, so that *φ*(*̅x̅*̅) *= φ̂*(∥*̅x̅*̅∥), or some other fixed point *̅c̅*̅, called a center (and/or, in particular active, control point), so that φ(*̅x̅*̅) = *φ̂*(∥*x̅ - c̅*∥)*.* Any function *φ,* that satisfies the property *φ*(*̅x̅*̅) = *φ̂*(∥*̅x̅*̅∥) is a radial function. The distance is usually Euclidean distance, although other metrics may be, and/or are sometimes, used. They are often used as a collection {*φₖ*}*ₖ* which forms a basis for some function space of interest.

The deformable registration function may be spline-based, in particular B-spline-based. A spline is generally a function defined piecewise by polynomials.

A B-spline or basis spline is generally a spline function that has minimal support with respect to a given degree, smoothness, and/or domain partition. Any spline function of given degree can be expressed as a linear combination of B-splines of that degree.

Alternatively or in addition, an example of RBFs may comprise a bump function, ${φ}_{i , α} \left(\vec{x}\right) = \left\{\begin{matrix}exp \left(\frac{1}{{\left‖\vec{x}-{\vec{c}}_{i}\right‖}^{2} - {s}_{α}^{2}}\right) , & if \left‖\vec{x}-{\vec{c}}_{i}\right‖ < {s}_{α} \\ 0 , & if \left‖\vec{x}-{\vec{c}}_{i}\right‖ \geq {s}_{α}\end{matrix}\right. ,$ with center point *̅c̅*̅ᵢ and width *s_{α}.*

In the following, the arrows on top of the vectors are often omitted. Alternatively or in addition, the basis functions for a plurality of *N* control points *c*₁, ··· *, c_{N}* are often denoted as *̅U̅*̅(*x̅, s_{α}*)= $\left[{φ}_{1 , α}\left(\vec{x}\right),⋯,{φ}_{N , α}\left(\vec{x}\right)\right] ∈ {ℝ}^{1 \times N}$. Omitting the arrows on top of the vectors leads to the alternative notation *U(x, s_{α}*) for the basis functions of the *N* control points with supports (and/or widths) *s_{α}.*

The plurality of active control points may vary throughout the nested iterations. Alternatively or in addition, the active control points may be inhomogeneously distributed over the first image and/or over the second image. The active control points may in particular be densely distributed within areas of misalignment (and/or areas of dissimilarity) of the first image and the (in particular deformed) second image. Alternatively or in addition, the (in particular active) control points may be sparse (and/or absent) in areas, where the first image and the (in particular deformed) second image are well aligned (and/or similar).

An initial number of active control points may be of the order of 1,000, or more.

The plurality of active control points may be a sparse set. Alternatively or in addition, a control point need not be located at a center of an image voxel.

For any newly selected active control point, a basis function (e.g., in terms of shape and/or width of support) may be selected. In an example, each basis function may be identical (e.g., the bump function) up to different width of support.

Basis functions may be determined before performing the method (also: before running the application). In some examples, a user may have selected (e.g., by means of a manual input on a user interface, UI, such as a graphical user interface, GUI) which basis functions will be used, and only the user-selected basis functions are selected during the entire optimization process, and/or for determining the deformable registration. Thus, which basis function or basis functions to use may be up to a user to define once prior to execution of the inventive technique.

The inventive technique allows the estimation of deformations (and/or of the deformable registration function) and inverse deformations (and/or the inverse of the deformable registration function) simultaneously. Alternatively or in addition, the inventive technique may only involve one set of active control points regardless of the output mapping directions (e.g., second medical image to first medical image, or vice versa). The control points may, e.g., be defined in the domain of the second medical image, and the initial transformation (and/or the initial deformation) may be used to map them to the domain of the first medical image, or vice versa. In other words, it does not matter in which domain the control points are defined for the inventive technique to work. The control points can be mapped between the two domains using the initial transformation (and/or initial deformation). In other examples, the (in particular active) control points are mapped, at the beginning of each optimization iteration, such as for a next level of spatial resolution, between the two domains using the up to then determined (and/or refined) transformation (and/or deformation).

The nested iterations may comprise a first type of iteration for optimizing the deformation parameters at a predetermined level of spatial resolution (also: multiresolution level) of the first image and the second image. At the first (and/or coarsest granularity) level of spatial resolution, the deformation parameters may be initialized by the deformable registration function being the identity function (and/or a global scaling, in case the second image differs in size from the first image).

E.g., for a deformable registration function having an affine transformation, an offset and warp coefficients (also: warping coefficients, and/or warp factors) as deformation parameters, the initial values may correspond to the identity matrix as affine transformation matrix (briefly also: affine matrix), zero offset and vanishing warp coefficients.

The nested iterations may comprise a second type of iteration by moving from the coarsest level of spatial resolution (also: coarsest granularity) to finer levels of spatial resolution (also: finer granularity) until a predetermined finest level of spatial resolution (also: finest granularity, e.g., corresponding to voxels or pixels of the first image and/or of the second image) is reached.

The (e.g., consecutive) levels of spatial resolution may comprise a multiplicative factor of spatial resolution (e.g., a factor of 2 per image direction, a factor of 2³=8 for 3D images, and/or a factor of 2²=4 for 2D images), e.g., in terms of a number of voxels per unit volume (or a number of pixels per unit area) per predetermined level of spatial resolution.

The optimization of the deformation parameters may, at each following finer level of spatial resolution, be initialized with the optimization result from the previous coarser level of spatial resolution.

For previously active control points that turned inactive, the optimization result from the last level of spatial resolution of the warp coefficient (and/or of the width) of the basis function may correspond to the final (and/or frozen) result.

New active control points may be introduced, for which new warp coefficients (and/or widths) may be optimized.

The iterative optimization of the set of deformation parameters at a predetermined level of spatial resolution may end (also: stop and/or terminate) if no active control points, and/or no (e.g., unit) areas with image dissimilarity exceeding the (in particular level-specific) predefined image dissimilarity threshold value, exist anymore.

The level-specificity of the predefined image dissimilarity threshold value may mean that as the level of spatial resolution becomes finer, the requirement on the image similarity increases.

Alternatively or in addition, the predefined image dissimilarity threshold value may decrease as the level of spatial resolution increases. Thereby, image regions that were sufficiently similar at a coarse level of spatial resolution (e.g., after the optimization of the deformation parameters for a selected plurality of active control points) may have led to turning some formerly active control points inactive. By decreasing the predefined image dissimilarity threshold value at the finer level of spatial resolution, it may be necessary to select new active control points for refining the deformable registration function.

Alternatively or in addition, the iterative optimization may end if the number of iterations hits a predefined maximum number of iterations, and/or if one or more stop criteria have been satisfied. The one or more stop criteria may include a minimum change (and/or a change below a predetermined threshold value from one step to the next within the iteration) in the magnitude of the derivative of the objective function. Alternatively or in addition, an objective function value may hit a certain threshold (e.g., close to 0 for minimization). Further alternatively or in addition, a minimum change (and/or a change below a predetermined threshold value) in objective function values per iteration may be reached, which may imply convergence.

A unit volume (or unit area) at any given level of spatial resolution (and/or granularity) may be provided in terms of a spatial extension and/or a number of voxels (or pixels). The finest (and/or final) level may correspond to one (or a few) voxels (or pixels) and/or the smallest spatial extension of the unit volume (or unit area). Alternatively or in addition, the coarsest (in particular the initial) level may correspond to a large number of voxels (or pixels) and/or a large spatial extension of the unit volume (or unit area).

Active control points may be adaptively selected, and/or may be selected according to the nested iterations. In one embodiment, the plurality of active control points is selected (e.g., independently) at each level of spatial resolution. In another embodiment, the plurality of active control points is selected (e.g., independently) for each iterative optimization of the deformation parameters at a predetermined level of spatial resolution.

The selected plurality of active control points may be iteratively reduced. Alternatively or in addition, an active control point may be defined by the local value of the image dissimilarity being at least equal to (or exceeding) the predefined image dissimilarity threshold value. When performing the nested iterations (e.g., at a predetermined level of spatial resolution), the local value of the image dissimilarity may decrease as the deformation parameters are optimized, leading to formerly active control points becoming inactive. The inactive control points may be discarded and/or sorted out (also: removed) from the further steps (e.g., the further deformation parameter optimizing by considering derivatives) of the nested iteration. By the discarding of the inactive control points, a significant amount of processing power may be saved. It is noted here that discarding from the future optimization amounts to retaining the latest optimized value of the deformation parameters (e.g., the warp coefficient in relation to the width of the basis function) at the by now inactive control point in a shared memory. Alternatively or in addition, storing the control points and deformation parameters in a shared memory may reduce storage space needed for performing the optimization, in particular when making use of parallel computing for different control points. The different control points may, e.g., be spaced apart by a distance larger than the sum of their supports. In such a case, the deformable registration at one control point is independent of the deformable registration at the other control point.

The predefined image dissimilarity threshold value may depend on the level of spatial resolution. E.g., the predefined image dissimilarity threshold value may decrease as the level of the spatial resolution increases. Alternatively or in addition, a plurality of active control points may be (e.g., independently) selected for each predetermined level of spatial resolution. For example, the active control points, which were selected based on a first (and/or initial, and/or largest) predefined image dissimilarity threshold value may have become inactive at the end of the iterative optimization at the first (and/or coarsest) level of spatial resolution. At the next (in particular finer) level of spatial resolution, a second (and/or lower) predefined image dissimilarity threshold value may lead to formerly active control points being activated again, and/or to newly selected active control points.

The first medical image and the second medical image may be acquired by means of independently selected medical imaging modalities.

By enabling to register images from different medical imaging modalities, it is possible to select the most appropriate modalities for their functionalities (e.g., a CT or MRT image acquisition for treatment planning, and a real-time image acquisition by means of CT, X-ray imaging or ultrasound during the treatment). Alternatively or in addition, not requiring the first image and the second image to be acquired by means of the same medical imaging modality facilitates transversal studies of diverse patients, and/or longitudinal studies of a patient, with medical images acquired by the at that time available scanner. This allows in particular for comparing newer images acquired by means of the most modern scanners with older images acquired by means of at that time established and/or available scanners.

A CT scan can for example be acquired in a few seconds. The time between the acquisition of a CT scan for treatment preparation and the start of treatment can vary considerably, e.g., be in the range of hours, days, and/or weeks. Hence, not the time needed for an image acquisition is typically the limiting factor, but the time from image availability to clinical decision making is critical. If information from prior imaging needs to be considered, it can be essential to make this information reliably available in an "anatomy of the day" and, depending on the clinical task, this may need to happen fast.

It is not necessarily a main objective to compare older hardware with newer. The quality of the images acquired throughout the clinical pathway may depend on the acquisition technique and space and/or time restrictions, e.g., an MRT acquisition prior to the treatment may be performed, but in the therapy room, only orthogonal X-rays (e.g., for acquiring the first medical image) may be available due to space limitations and/or because the patient cannot be moved. Hence, it may be advantageous to try to get the better 3D information prior to treatment (e.g., as the second medical image) into the treatment room by using (in particular deformable) registration.

To enable the comparing and/or registering of images (in particular from different medical imaging modalities), a modality-independent neighbourhood descriptor (MIND) may be used. The MIND may comprise, for each voxel (or pixel) in an image, a vector of real numbers describing a local structure surrounding that voxel (or pixel). The vector may, e.g., comprise a greyscale per voxel (or pixel), and/or a voxel location (or pixel location). The MIND may be determined independently for the (e.g., first and second) images to be (in particular deformably) registered. MIND is, e.g., described by Mattias P. Heinrich et al. in [8], which is incorporated herein by reference.

Alternatively or in addition, for comparing and/or registering of images (in particular from different medical imaging modalities), a segmentation of each image may be used. E.g., a result of the segmentation may be a multi-mask comprising (e.g., binary) segmentation masks for different organs. The (e.g., deformable) registration of the (e.g., first and second) images may be initialized based on mapping matching segmentation masks onto each other.

The first medical image and the second medical image may be acquired by means of the same medical imaging modality.

Determining a dissimilarity and/or performing the registration procedure may be simpler for images that are similar, e.g., in terms of their image parameters (e.g., frequencies, opacities, image contrasts and/or colors) and/or image resolution.

The step of iteratively optimizing the set of deformation parameters may be performed using parallel computing. The parallel computing may comprise distributing the iterative optimizing according to the selected active control points across parallel computing resources.

The deformable registration function may comprise a sum over basis functions with localized and/or compact support. Alternatively or in addition, the basis functions may be localized at different (e.g., active) control points and not (or at least not significantly) influence the values of the deformable registration function at the other (e.g., active) control points. Optimizing the deformation parameters, in particular warp coefficients (also: weight coefficients) associated with basis functions, can thus be advantageously done independently for each active control point. Alternatively or in addition, the independent optimization at different active control points can be implemented in parallel on different processing units (e.g., central processing units, CPUs, graphics processing units, GPUs, and/or different cores of a CPU and/or of a GPU). Thereby, the optimization process can optimally use existing computing hardware, and/or the optimization process can be sped up, e.g., by determining multiple warp coefficients simultaneously.

The plurality of active control points may be adaptively selected per iterative optimization step and/or after a predetermined number of iterative optimization steps, in particular at a predetermined level of spatial resolution. Alternatively or in addition, the plurality of active control points may be adaptively selected per predetermined level of spatial resolution.

After each iterative optimization step, or after a predetermined number of iterative optimization steps (e.g., after every second or every fifth iterative optimization step), the image dissimilarity of the deformed second medical image and the first medical image may be re-evaluated (and/or determined again). A previously active control point may then be set inactive, if the image dissimilarity in the region, where the control point is located, has fallen below the predefined image dissimilarity threshold value. This may in particular be due to the warp coefficient (and/or the width, also: width of support) of the corresponding basis function being changed (and/or optimized).

In the iterative optimization at a predetermined level of spatial resolution, the number of active control points may decrease with the number of iterative optimization steps performed.

Alternatively or in addition, upon proceeding from one (e.g., previous) predetermined level of spatial resolution (e.g., with coarse granularity) to the next predetermined level of spatial resolution (e.g., with the next finer granularity), the plurality of active control points may be reset and/or newly selected. The new selection may in particular be performed based on a lower predefined image dissimilarity threshold value of the next predetermined level of spatial resolution as compared to the previous one.

The plurality of active control points may thus be selected as necessary, further improving the use of computing resources (e.g., processing resources and/or memory resources). Alternatively or in addition, whenever a control point is set inactive, a related deformation parameter (e.g., a warp coefficient and/or width of support) need not further be optimized, freeing up processing resources and/or not requiring any further update in a shared memory.

Alternatively of in addition, control points, where a basis function has support, may be queried according to a spatial tree and/or a graph structure. The basis functions only need to be evaluated simultaneously for those control points. An optimization process at one point may thus comprise more than one warp coefficient and/ or more than one width of support. This situation may in particular occur if a distance of two control points is less then the sum over the the corresponding widths of support.

Selecting the plurality of active control points may be based on point sampling. Optionally, performing the iteration over (in particular increasingly finer) levels of spatial resolution may comprise repeating point sampling.

Point sampling may be a method of determining appropriate trees to sample within a sampling or cruising system. Alternatively or in addition, point sampling may be a method to distribute active control points across a (e.g., connected) region, where the predefined image dissimilarity threshold value is exceeded (and/or at least reached).

A number of points passing the control-point criteria (e.g., exhibiting high dissimilarity values) may be larger than a number of control points specified (e.g., by a user). In this case, active control points need to be sampled from all control-point candidates. Random sampling or sampling using farthest-point sampling approaches may be used, for example.

The deformable registration function may comprise a spline function, in particular a B-spline function. Alternatively or in addition, the deformable registration function may comprise a sum over basis functions, in particular radial basis functions (RBFs), and more particularly compactly-supported RBFs (CSRBFs).

The deformable registration function may be locally expressible as a sum over polynomials, such as a spline function or a sum over (in particular radial) basis functions. Advantageously, the deformable registration function may be expandable as a sum over basis functions having only support in a local neighbourhood of a center and/or (e.g., active) control point. E.g., the deformable registration function may comprise a B-spline function and/or may be expandable in terms of CSRBFs. Thereby, parallel computing for (in particular simultaneously) determining the deformation parameters in relation to different active control points may be facilitated.

The set of deformation parameters may comprise an affine transformation matrix, an offset, a set of warp coefficients, and/or a set of associated widths of support.

The set of warp coefficients may also be denoted as warp matrix. Alternatively or in addition, the set of warp coefficients may correspond to coefficients of a warp function, which may be expandable in terms of RBFs. Any spatial (and/or non-universal) dependence may be encoded in an affine transformation term, which may be identical for all points (and/or, volumes, areas, and/or regions) of the second image, and a sum over RBFs, which each depend on an input point and, in case of a CSRBF, advantageously are each only non-vanishing in a local neighbourhood (such as parameterized by a width of support) around a control point. Thereby, parallelization of the parameter optimization, in particular of the warp coefficients and/or the widths of support, is facilitated.

In some embodiments, the basis functions, in particular CSRBFs to be used as well as a (e.g., discrete) set of widths of support may be selected by a used before performing the deformation parameter optimization. Alternatively or in addition, the width of support need not be a parameter to be optimized.

The set of deformation parameters (e.g., including the widths of support, even if pre-selected and/or not optimized according to the inventive technique) and/or the selected plurality of control points (in particular including active control points and/or inactive control points) may be stored in a shared memory.

Alternatively or in addition, a set of input points and a set of output points of the deformable registration function may be stored in the shared memory.

By using a shared memory, each quantity only needs to be stored once. Alternatively or in addition, any optimization and/or update performed in one parallel computation becomes (in particular immediately) accessible to all other optimization processes.

In the shared memory, input points and output points may be stored. The input points may be points used in determining (e.g., calculating) terms in the objective functions (particularly, point similarity and/or penalty functions). The input points may be used in every iteration to compute the objective function and to update the deformation parameters (and/or for optimization update function). Alternatively or in addition, the input points may be used to determine the image dissimilarity, e.g., at each level of the spatial iteration. The input points may be, e.g., uniformly, distributed over one of the medical images (e.g., the second or first medical image).

The input points may be generally different from the control points. The control points may be part of the deformation function, in particular acting as center points of basis functions. Alternatively or in addition, input points may be transformed by the deformation function (which may be defined based only on control points since control points define the deformation), resulting in output points. The output points may be used together with the corresponding input points to determine (in particular calculate) the objective function and deformation parameter update.

Input points and control points are, according to the inventive technique, advantageously stored in the shared memory throughout the deformation (and/or registration) process. Output points are, according to the inventive technique, also stored in the shared memory, but the values may change, such as per iteration.

The plurality of active control points may be selected for one image, e.g., for the second medical image. Alternatively or in addition, a plurality of active control points of the other image, e.g., of the first medical image, may be determined by applying the deformable registration function (and/or its inverse). The mapping of the (in particular active) control points between the first image and the second image may, in particular at the initial (and/or coarsest) level of resolution be performed by using the initial values of the deformation parameters. In some embodiments, the mapping of the (in particular active) control points between the first image and the second image may, at any given finer level of spatial resolution, be performed using the optimized values of the deformation parameters from the previous (and/or next coarser) level of spatial resolution.

Supports of radial basis functions may correspond to scalar values, defining the deformation function together with the control points. Further defining the deformation function may be affine transformation, the offset, and/or the warp coefficients.

The objective function may comprise a measure of a point cloud similarity and/or a measure of an image similarity.

The point cloud similarity may replace a discrete point similarity by a continuous, in particular analytic function.

The objective function may be a sum over two or more terms. E.g., the point cloud similarity and/or the image similarity may be measured by various analytic functions, such as a mean squared error (MSE), a mean distance (MD, also denoted as L2 distance) and/or another function, such as a Gaussian mixture of a discrete point set, the deformable registration function, and/or its inverse.

A dissimilarity may be the negative (and/or the inverse) of a similarity. E.g., the value zero for a MSE or MD may correspond to maximal similarity and/or minimal dissimilarity.

The inverse of the deformable registration function may deform the first image to the second image. The inverse of the deformable registration function (and/or its deformation parameters) may be fully determined by the deformable registration function (and/or the corresponding deformation parameters, e.g., for preset basis functions and/or sets of width of support).

Optimizing the objective function may thus correspond to maximizing a similarity between the images (and/or points on the images). Alternatively or in addition, optimizing the objective function may correspond to minimizing a dissimilarity between the images (and/or points on the images).

Optimizing the objective function may comprise imposing a set of physical constraints comprising at least one physical constraint. Optionally, imposing the set of physical constraints may comprise including corresponding physical constraint terms in the objective function. Alternatively or in addition, imposing the set of physical constraints may comprise using corresponding Lagrange multipliers for the optimization.

A physical constraint (also: penalty and/or regularisation term) can ensure that, e.g., rigid structures, such as bones, are not deformed in a physically unfeasible way. Alternatively or in addition, constraints on relative locations of different tissue types and/or different organs, and/or their compressibility (and/or change in volume, e.g., due to breathing and/or a phase in the patient's cardiac cycle) can be imposed on the deformable registration. Thereby, an accuracy and/or reliability of the deformable registration can be improved.

Each physical constraint may be imposed as one or more (e.g., two, one for the deformable registration function and another one for its inverse) terms summed to the constraint-free objective function. Alternatively or in addition, the method of Lagrange multipliers may be used to modify the objective function.

Optimizing the objective function may be symmetric in its dependence on the deformable registration function and its inverse. Alternatively or in addition, the optimization (and/or the nested iterations) may be independent, e.g., at least in the result, of which image is interpreted as still image and which image is interpreted as moving image, and/or on which of the two images the deformation is performed.

The set of physical constraints (in particular with at least one physical constraint) may comprise an affinity or bending energy constraint, a landmark matching, a sliding motion constraint, a bone rigidity constraint, a tissue incompressibility constraint, and/or a topology preservation constraint.

The physical constraints can improve well-posedness, an optimization stability, and/or a physical plausibility of deformation outcomes.

The affinity or bending energy constraint may also be denoted as smoothness constraint.

The landmark matching may make use of recognizable structure (and/or landmarks) in both (e.g., first and second) images. Thereby, a geometric accuracy of the deformable registration may be improved. The landmarks may, e.g., be provided by a segmentation of the first image and of the second image. E.g., in case it is known that the patient has an implant (e.g., a stent), the implant may serve as landmark for registering the second image to the first image, or vice versa. Alternative examples of landmarks include anatomical structures, such as a cardiac valve.

The sliding motion constraint may use surface normal vectors at surface points to model organ interfaces. E.g., the lung and/or bladder may be moving according to predetermined rules relative to adjacent organs.

The bone rigidity constraint may impose a rigid motion and/or an orthogonal property of a rotation matrix in relation to bone structures within the image. E.g., the patient's spine may be assumed to be not moving.

The tissue incompressibility constraint may impose limits on compression and/or expansion depending on the (e.g., soft) tissue type. The tissue incompressibility constraint may depend on the type of tissue (and/or the organ to which the tissue belongs).

The topology preservation constraint may also be denoted as inverse consistency constraint.

A dissimilarity of an image, or of a point cloud, may be determined by a mean distance (MD), a mean squared error (MSE), gradient information, and/or combined mutual information.

The MD may be minimized for maximal (e.g., image and/or point cloud) similarity.

Alternatively or in addition, the MSE may be minimized for maximal (e.g., image and/or point cloud) similarity. The MSE may be suitable if the first image and the second image are acquired by means of the same medical imaging modality.

The gradient information may be maximized for maximal (e.g., image and/or point cloud) similarity. The gradient information may be independent of the medical imaging modalities. In particular, the gradient information may be determined if the medical imaging modalities of the first and second image are independently selected.

The combined mutual information may be maximized for maximal image similarity.

Alternatively or in addition, a point cloud dissimilarity may be based on a mixture of parameters, in particular (e.g., density) weights, widths and/or centers of radial functions (e.g., Gaussians). The (e.g., density) weights may be low if an uncertainty at a point is high, and vice versa.

The inventive technique may make use of first derivatives at any point on the second image (and/or on the first image). The basis functions, and also the (in particular first) derivatives of the basis functions may each take a constant value at a predetermined point. It is thus only necessary to compute the value of the basis function and the value of each derivative once per point on the image. The set of values per point can then be stored in the shared memory and used for the optimization of the deformation parameters.

In alternative examples, second order derivatives (and/or Hessians) of the deformable registration function, and/or of the objective function, may be used.

Receiving the first medical image and receiving the second medical image may comprise receiving a segmentation mask, in particular a multi-mask, in relation to the first medical image and in relation to the second medical image, respectively.

Performing the nested iterations may comprise a step of receiving an indication of a downsampling factor and/or of an initial predetermined level of spatial resolution.

Alternatively or in addition, performing the nested iterations may further comprise a step of downsampling the first medical image, and/or the second medical image, to arrive at the initial predetermined level of spatial resolution. Alternatively or in addition, performing the nested iterations may further comprise a step of performing an upsampling of the first medical image, and/or the second medical image, to arrive at the further predetermined level of spatial resolution.

To perform the nested iterations, the indication of the downsampling factor (e.g., 2" for a number n=3,4 or 5) may be received. The downsampling factor may be different (and/or independent) for each spatial direction. The downsampling factor may be a maximal downsampling factor to arrive at the initial predetermined level of spatial resolution.

For each finer level of spatial resolution, an upsampling by a predetermined factor (e.g., 2¹ for one spatial direction) may be performed. The upsampling may be performed independently for each spatial direction.

Thereby, each level of spatial resolution may be determined, e.g., until a finest (and/or final) spatial resolution of the first medical image and/or of the second medical image is reached.

The method may be used for support of an image-guided intervention, for treatment monitoring, for image comparisons, and/or for radiotherapy planning.

The method may be performed by a computing device.

The (in particular computer-implemented) method may be executed by a plurality of processors (e.g., 10 CPUs or 10 GPUs), each in data communication with a shared memory. The processors and memory may be realised as hardware of the computing device. The processors may work independently and/or in parallel on different threads.

As to a device aspect, a computing device for providing a deformable registration result of a second medical image to a first medical image is provided. The computing device comprises a first input interface configured for receiving a first medical image, the first medical image comprising at least one anatomical structure. The computing device further comprises a second input interface configured for receiving a second medical image. The second medical image comprises the at least one anatomical structure. The computing device further comprises a nested iterations module configured for performing nested iterations. The nested iterations comprise an iteration over levels of spatial resolution with increasing spatial resolution. The nested iterations further comprise, at each level of spatial resolution, an iteration regarding a set of deformation parameters of an analytic deformable registration function for registering a deformation of the received second medical image to the received first medical image.

The nested iterations may comprise, for a predetermined level of spatial resolution, determining an image dissimilarity between the received first medical image and the received second medical image. The image dissimilarity may be determined per unit volume (or unit area) related to the predetermined level of spatial resolution. The nested iterations may further comprise, for the predetermined level of spatial resolution, selecting a plurality of active control points. The active control points may be located in regions of the first medical image and/or of the second medical image, in which the determined image dissimilarity exceeds (or at least equals to) a (in particular level-specific) predefined image dissimilarity threshold value. The nested iterations may further comprise, for the predetermined level of spatial resolution, iteratively optimizing the set of deformation parameters by iteratively optimizing an objective function. The objective function may be a differentiable function of the analytic deformable registration function. The analytic deformable registration function may comprise a sum over analytic functions centered at the selected active control points.

The iteration over levels of spatial resolution may comprise proceeding to a further predetermined level of spatial resolution, which has a finer spatial resolution of spatial resolution and/or a smaller unit volume (or unit area) than the previous predetermined level of spatial resolution, and repeating the steps of determining an image dissimilarity, selecting a plurality of active control points, and iteratively optimizing the set of deformation parameters at the further predetermined level of spatial resolution. The iteration over levels of spatial resolution may further comprise terminating the nested iterations if (or when), for a predetermined level of spatial resolution with finest spatial resolution and/or with a predetermined smallest size of unit volume (or unit area), the set of deformation parameters has been iteratively optimized.

The computing device further comprises an output interface configured for providing a deformable registration result of registering the second medical image to the first medical image. The deformable registration result comprises utilizing the set of deformation parameters in the deformable registration function after the optimizing terminated.

The nested iterations module may comprise a plurality of processors, which are suitable for parallel computing.

The computing device may be configured to perform any one of the steps, and/or may comprise any one of the features, disclosed in the context of the method aspect.

As to a further aspect, a computer program product is provided comprising program elements which induce a computing device to carry out the steps of the method for providing a deformable registration result of a second medical image to a first medical image according to the method aspect, when the program elements are loaded into a memory of the computing device.

As to a still further aspect, a computer-readable medium is provided on which program elements are stored that can be read and executed by a computing device, in order to perform steps of the method for providing a deformable registration result of a second medical image to a first medical image according to the method aspect, when the program elements are executed by the computing device.

The properties, features and advantages of this invention described above, as well as the manner they are achieved, become clearer and more understandable in the light of the following description and embodiments, which will be described in more detail in the context of the drawings.

This following description does not limit the invention on the contained embodiments. Same components or parts can be labeled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

- Fig. 1: is a flow chart of a method for providing a deformable registration result of a second medical image to a first medical image according to a preferred embodiment of the present invention;
- Fig. 2: is an overview of the structure and architecture for providing a deformable registration result of a second medical image to a first medical image according to a preferred embodiment of the present invention;
- Fig. 3: schematically illustrates a computing architecture for performing parallel computing using a shared memory. The computing device may be the device of Fig. 2;
- Fig. 4: schematically shows data stored in the shared memory of the computing device of Fig. 2 and/or Fig. 3, including input points and output points used for determining an image dissimilarity, control points and deformation parameters;
- Figs. 5(a), (b), (c) and (d): show an example of an adaptive control point distribution overlayed on a MIND map and on the first medical image for two different levels of spatial resolution; and
- Fig. 6: shows an example of a physically constrained tissue dependent deformation according to the inventive technique, in particular using the method of Fig. 1, versus conventional techniques lacking consistency constraints.

Any reference signs in the claims should not be construed as limiting the scope.

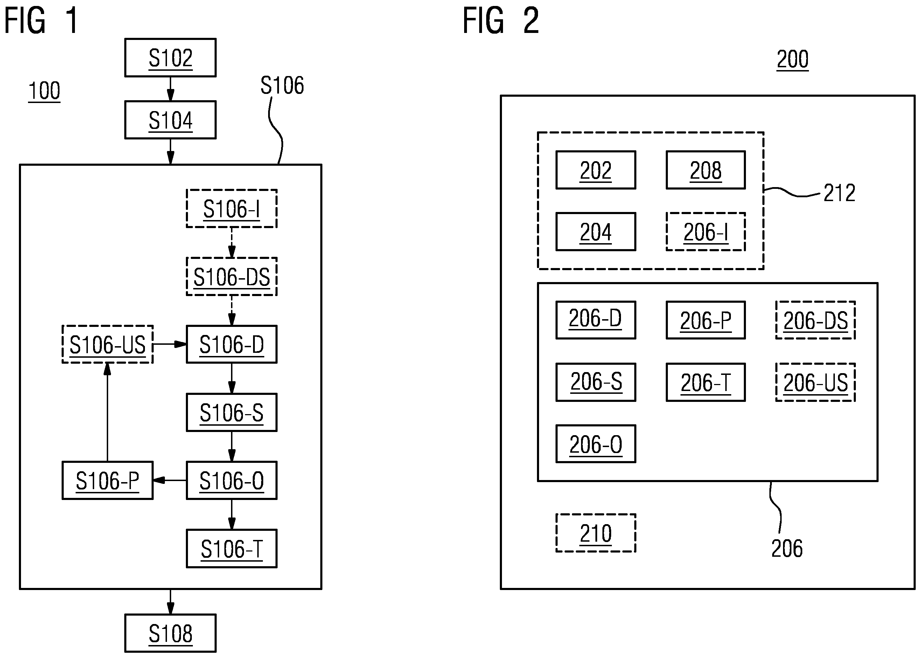

Fig. 1 schematically illustrates an exemplary flowchart for a (in particular computer-implemented) method for providing a deformable registration result of a second medical image to a first medical image. The method is generally referred to by the reference sign 100.

The method 100 comprises a step S102 of receiving a first medical image. The first medical image comprises at least one anatomical structure. The method 100 further comprises a step S104 of receiving a second medical image. The second medical image comprises the (e.g., same and/or analogous) at least one anatomical structure. The method 100 further comprises a step S106 of performing nested iterations. The nested iterations comprise an iteration over levels of spatial resolution with increasing spatial resolution. The nested iterations further comprise, at each level of spatial resolution, an iteration regarding a set of deformation parameters of an analytic deformable registration function for registering a deformation of the received S104 second medical image to the received S102 first medical image.

The nested iterations S106 in the example of Fig. 1 comprise, for a predetermined level of spatial resolution, a sub-step S106-D of determining an image dissimilarity between the received S102 first medical image and the received S104 second medical image. The image dissimilarity is determined per unit volume (or unit area) related to the predetermined level of spatial resolution. The nested iterations S106 further comprise, for the predetermined level of spatial resolution, a sub-step S106-S of selecting a plurality of active control points. The active control points are located in regions of the first medical image and/or of the second medical image, in which the determined S106-D image dissimilarity exceeds (or at least equals to) a (in particular level-specific) predefined image dissimilarity threshold value. The nested iterations S106 further comprise, for the predetermined level of spatial resolution, a sub-step S106-O of iteratively optimizing the set of deformation parameters by iteratively optimizing an objective function. The objective function is a differentiable function of the analytic deformable registration function. The analytic deformable registration function comprises a sum over analytic functions centered at the selected S106-S active control points.

The iteration over levels of spatial resolution in Fig. 1 comprises a substep S106-P of proceeding to a further predetermined level of spatial resolution, which has a finer level of spatial resolution and/or a smaller unit volume (or unit area) than the previous predetermined level of spatial resolution, and repeating the steps of determining S106-D an image dissimilarity, selecting S106-S a plurality of active control points, and iteratively optimizing S106-O the set of deformation parameters at the further predetermined level of spatial resolution. The iteration over levels of spatial resolution further comprises a substep S106-T of terminating the nested iterations if, for a predetermined level of spatial resolution with finest spatial resolution and/or with a predetermined smallest size of unit volume (or unit area), the set of deformation parameters has iteratively optimized S106-O (and in particular is not further optimized when repeating the steps S106-D, S106-S, and/or S106-O).

The method 100 further comprises a step S108 of providing a deformable registration result of registering the second medical image to the first medical image. The deformable registration result comprises utilizing the, after the optimizing terminated S106-T, set of deformation parameters in the deformable registration function.

The plurality of active control points may be adaptively selected S106-S in each iterative optimization step and/or after a predetermined number of iterative optimization steps, in particular at a predetermined level of spatial resolution. Alternatively or in addition, the plurality of active control points may be adaptively selected S106-S at each predetermined level of spatial resolution.

Selecting S106-S the plurality of active control points may be based on point sampling. Optionally, performing the iteration over (in particular finer) levels of spatial resolution comprises repeating point sampling.

Performing S106 the nested iterations may further comprise a substep S106-I of receiving an indication of a downsampling factor and/or of an initial predetermined level of spatial resolution. Performing S106 the nested iterations may further comprise a substep S106-DS of downsampling the first medical image and/or the second medical image to arrive at the initial predetermined level of spatial resolution. Performing S106 the nested iterations may further comprise a substep S106-US of performing an upsampling of the first medical image and/or the second medical image to arrive at the further predetermined level of spatial resolution.

Fig. 2 schematically illustrates an exemplary architecture of a computing device for providing a deformable registration result of a second medical image to a first medical image. The computing device is generally referred to by the reference sign 200.

The computing device 200 comprises a first input interface 202 configured for receiving a first medical image. The first medical image comprises at least one anatomical structure. The computing device 200 further comprises a second input interface 204 configured for receiving a second medical image. The second medical image comprises the (e.g., same or analogous) at least one anatomical structure. The computing device 200 further comprises a nested iterations module 206 configured for performing nested iterations. The nested iterations comprise an iteration over levels of spatial resolution with increasing spatial resolution. The nested iterations further comprise, at each level of spatial resolution, an iteration regarding a set of deformation parameters of an analytic deformable registration function for registering a deformation of the received second medical image to the received first medical image.

The nested iterations module 206 may comprise an image dissimilarity determining submodule 206-D, which is configured for determining an image dissimilarity between the received first medical image and the received second medical image. The image dissimilarity is determined per unit volume (or unit area) related to the predetermined level of spatial resolution.

The nested iterations module 206 may further comprise an active control point selecting submodule 206-S, which is configured for selecting a plurality of active control points. The active control points may be located in regions of the first medical image and/or of the second medical image, in which the determined image dissimilarity exceeds (or at least equals to) a, in particular level-specific, predefined image dissimilarity threshold value.

The nested iterations module 206 may further comprise an iteratively optimizing submodule 206-O, which is configured for iteratively optimizing the set of deformation parameters by iteratively optimizing an objective function. The objective function may be a differentiable function of the analytic deformable registration function. The analytic deformable registration function may comprise a sum over analytic functions centered at the selected active control points.

The iteration over levels of spatial resolution may comprise proceeding to a further predetermined level of spatial resolution, which has a finer spatial resolution of spatial resolution and/or a smaller unit volume (or unit area) than the previous predetermined level of spatial resolution, and repeating the steps of determining an image dissimilarity, selecting a plurality of active control points, and iteratively optimizing the set of deformation parameters at the further predetermined level of spatial resolution. A further proceeding submodule 206-P may be configured for the proceeding to the further predetermined level of spatial resolution.

The iteration over levels of spatial resolution may further comprise terminating the nested iterations if, for a predetermined level of spatial resolution with finest spatial resolution and/or with a predetermined smallest size of unit volume (or unit area), the set of deformation parameters is iteratively optimized. A terminating submodule 206-T may be configured for the terminating of the nested iterations.

The computing device 200 further comprises an output interface 208 configured for providing a deformable registration result of registering the second medical image to the first medical image. The deformable registration result comprises utilizing the, after the terminating optimized, set of deformation parameters in the deformable registration function.

The nested iterations module 206 may further comprise a downsampling factor indication reception submodule 206-I, which is configured for receiving an indication of a downsampling factor and/or of an initial predetermined level of spatial resolution.

The nested iterations module 206 may further comprise a downsampling submodule 206-DS, which is configured for downsampling the first medical image and/or the second medical image to arrive at the initial predetermined level of spatial resolution.

The nested iterations module 206 may further comprise an upsamling submodule 206-US, which is configured for performing an upsampling of the first medical image and/or the second medical image to arrive at the further predetermined level of spatial resolution.

Any module may alternatively be denoted as unit, and vice versa.

The computing device 200 may comprise an input-output (I/O) interface 212. The I/O interface may embody the first input interface 202, the second input interface 204, output interface 208 and/or the optional downsampling factor indication reception submodule 206-I.

The computing device 200 may comprise a set of, in particular, parallel processors 206, which may (e.g., each) embody the nested iterations module 206 (and/or any of its optional submodules 206-D; 206-S; 206-O; 206-P; 206-T; 206-DS; 206-US).

The computing device 200 may comprise a memory 210. The memory 210 may be a shared memory.

The computing device 200 may be configured for performing the method 100.

A system may comprise the computing device 200 and a database, such as a picture archiving and communication system (PACS) database, from which the first medical image and the second medical image may be received. Alternatively or in addition, the system may comprise a scanner, from which the first medical image and the second medical image may be received.

The system may be configured to perform the method 100.

The inventive technique (e.g., comprising the method 100, and/or the computing device 200) provides a fast implementation of a non-learning deformable registration optimizing a parametric transformation under various physical constraints. To allow to resolve large and/or varying motion while minimizing memory usage, the inventive technique estimates deformation based on a set of active control points, which may be determined on the fly based on the quality of image alignment in their local neighbourhood. Alternatively or in addition, to speed up the optimization, a derivative-based optimization with analytical gradients for all the terms in the objective functions is performed. This contrasts conventional learning approaches whose performance is currently constrained by CPU/GPU memory.

The inventive technique (e.g., comprising the method 100, and/or the computing device 200) focuses on speeding up non-learning iterative methods. Prior work introduces approaches to speed up non-parametric deformable registration methods (i.e., estimating displacement fields) involving at most smoothness constraints (e.g., diffusive regularization and diffeomorphic regularization) [2-6]. These approaches lack the ability to resolve large motion and sharp change of adjacent motion (in terms of magnitude and/or direction). They are also unable to model motion based on tissue properties (like all neural field and learning methods), i.e., causing deformation of bone and smooth coherence motion of otherwise discontinuous sliding interface. Unlike prior work, the inventive technique can speed up a constrained parametric-based method, exploiting the properties of parametric deformations, the parallel nature of the problem, and/or analytical calculation. The inventive technique can be implemented in both CPU and GPU using, for example, OpenMP for the former and CUDA for the latter.

The inventive technique (e.g., comprising the method 100, and/or the computing device 200) may alternatively be denoted as parallel computing for constrained parametric deformable registration with analytical derivatives and misalignment-based adaptive control points.

By the inventive technique, parametric deformable registration may be sped up. The approach may be applicable to spline-based deformation (such as using Bspline and/or radial basis functions), i.e., a deformation of the form: $ψ \left(x\right) = xA + t + {\sum }_{α} U \left(x, {s}_{α}\right) {W}_{α} ,$ (1) where $A ∈ {ℝ}^{d \times d}$ is an affine matrix, $t ∈ {ℝ}^{1 \times d}$ denotes an offset, and $U ∈ {ℝ}^{1 \times N}$ is a basis function with a support *s_{α}* ∈ 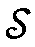 associated with *N* control points $C = {\left\{{c}_{i}\right\}}_{i = 1}^{N}$ and warp coefficients *W_{α}* ∈ ${ℝ}^{N \times d}$. The equation (1) covers various deformation models, including but not limited to a transformation involving a single warp coefficient matrix and a basis function with global support (i.e., *s_{α}* = ∞)*, ψ*(*x*) *= Ax + t + U*(*x*)*W,* and a transformation involving only deformation *ψ*(*x*) = *x + U*(*x*)*W* or *ψ*(*x*) *= U*(*x*)*W.* The proposed multiprocessing approach can be implemented in CPU (using e.g., OpenMP) or GPU (using e.g., CUDA).

In eq. (1), the input point *x* and output point *y = ψ*(*x*) are each written as a d-dimensional (with d=3 for 3D images and d=2 for 2D images) vectors, and the sum over basis functions at control points is written as product of matrices, i.e., ${\sum }_{α} U \left(x, {s}_{α}\right) {W}_{α} = {\sum }_{α} {\sum }_{i = 1}^{N} U \left(\left‖x-{c}_{i}\right‖, {s}_{α}\right) {W}_{αi}$ where *W_{αi}* is the i-th row of *W_{αi}*, for different radial basis functions *U* centered at the same control point *cᵢ* implicit in the summing over the index α for the supports *s_{α}* and warp coefficients *W_{αi}.*

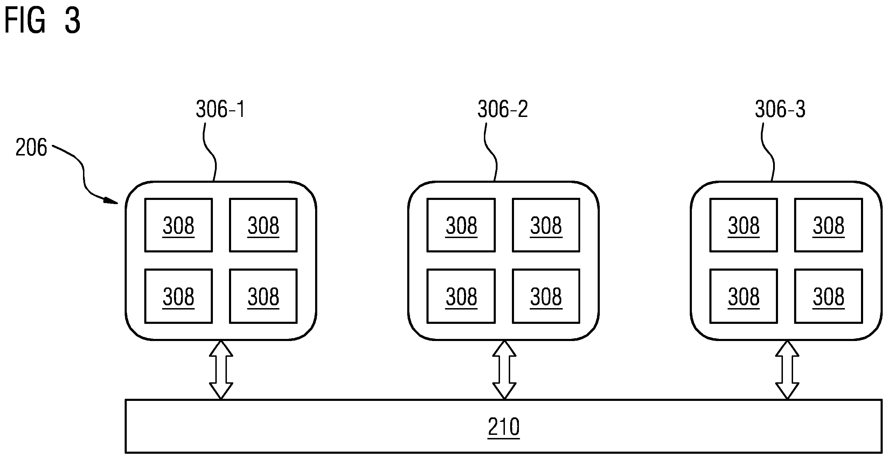

One can see that eq. (1) is independent of an (and/or is valid for any) input point $x ∈ {ℝ}^{1 \times d}$. This means that each input point *x* can be processed separately using different processing and/or computing nodes and/or cores (e.g., cores in CPU and/or GPU units). As an example, Fig. 3 schematically shows a basic diagram of a computing architecture found in typical computers nowadays. For illustrative purposes, the exemplary architecture in Fig. 3 shows three processors 306-1; 306-2; 306-3, each having four computing cores 308, in data connection with the shared memory 210

In the example of Fig. 3, for a conventional serial program where each point is processed sequentially, only a single process is executed using a single core 308 at a time, which is highly inefficient. Instead, according to the inventive technique one can exploit the hardware 206; 306-1; 306-2; 306-3 to speed up calculations by distributing the evaluation of eq. (1) for each point (e.g., x) to different processes (and/or cores 308), referred to as multiprocessing. Alternatively or in addition, multiprocessing may comprise distributing the computation for different control points *cᵢ* and/or (in particular compactly-supported) basis functions. An example is provided in Algorithm III.

The simplest way to transform a set of input point using eq. (1) in a multiprocessing scheme is to copy the deformation parameters *A, t,* 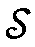 *,* and *W_{α}* for all *α* to all the processes involved in transforming the input point set. This means conventionally that each process must allocate a big memory space to store copies of *A, t,* 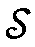 , and *W_{α}* for all *α,* which is highly redundant and inefficient and could be infeasible if any of the matrices are too big. Additionally, since *A, t,* and *W_{α}* for all *α* are parameters to be optimized in the deformable-registration optimization, their values are to be updated iteratively. Having the copies for each process requires the processes to communicate the parameter update values, which in turn, conventionally increases computation time. Thus, besides increased memory usage for redundant data, having separate copies of information for each process increases runtime.

In some embodiments, the widths of the supports are user-defined values that are input (e.g., by means of a UI, such as a GUI) before the deformable registration is executed.

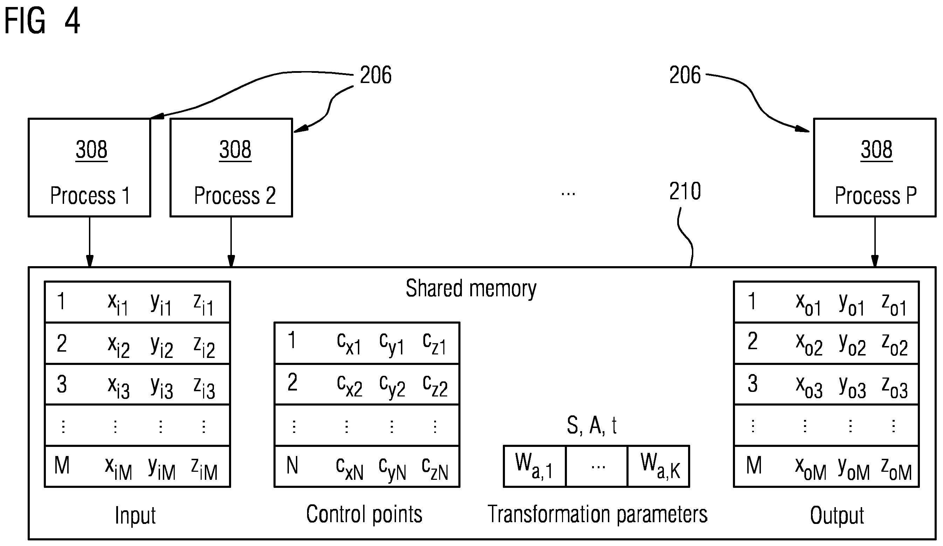

Alternately, *C,* 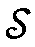 *, A, t,* and *W_{α}* can be stored in a shared memory since eq. (1) only reads (and/or uses) *C,* 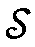 *, A, t,* and *W_{α}* (but not modify and/or write to). By storing *C*, 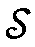 *, A, t,* and *W_{α}* in a shared memory, the inventive technique benefits from the reduced memory footprint required to transform a set of input points ${X}_{i} = {\left\{{x}_{ij}\right\}}_{j = 1}^{M}$, where the index *i* denotes "input". This way of processing follows a shared memory multiprocessing architecture, as exemplarily shown in Fig. 4, supported by various multiprocessing libraries (e.g., OpenMP, CUDA).

Fig. 4 schematically illustrates shared memory multiprocessing to transform multiple input points ${X}_{i} = {\left\{{x}_{ij}\right\}}_{j = 1}^{M}$ simultaneously to output points ${X}_{o} = {\left\{{x}_{oj}\right\}}_{j = 1}^{M}$ using eq. (1). It is noted that the indices i and *o* of the sets of points *Xᵢ* and *Xₒ* simply denote "input" and "output", respectively. For the deformation function being used to deformably register the second medical image to the first medical image, the set of input points *Xᵢ* corresponds to points within the original first medical image. Alternatively or in addition, the set of output points *Xₒ* corresponds to points within the second medical image, which is aimed for maximizing similarity with the first medical image, or vice versa.

In Fig. 4, 1... K are flattened indices to each *W_{α},* such as *K = N* × *d.* The i-th row and j-th column of *W_{α}* thus correspond to the flattened index *i* × *d + j.* Moreover, differently from the notation of *xᵢⱼ* as the j-th input vector *x̅ᵢⱼ* throughout the text, in Fig. 4, the coordinate entries of three-dimensional vectors (and/or d=3) (*xᵢⱼ, yᵢⱼ, zᵢⱼ*) *= x̅ᵢⱼ*, with similar notations for the output vectors and control points in Fig. 4. Algorithm I shows exemplary pseudocode for transforming multiple input points *Xᵢ* to output points ${X}_{o} = {\left\{{x}_{oj}\right\}}_{j = 1}^{M}$ simultaneously based on a shared memory multiprocessing architecture, such as depicted in Fig. 4. Although the exemplary Algorithm I assumes that multiprocessing is achieved through OpenMP (i.e., using #pragma omp parallel), other multiprocessing libraries such as CUDA can be used as well.

### Algorithm I: Spline-based transformation of multiple input points simultaneously

| | | |
|---|---|---|
| Input | Input points ( ${X}_{i} = {\left\{{x}_{ij}\right\}}_{j = 1}^{M}$) | |
| | Control points ( $C = {\left\{{c}_{i}\right\}}_{i = 1}^{N}$) | |
| | CSRBF support ( 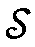 *=* {*s_{α}*}) | |
| | Offset (*t*) | |
| | Affine matrix (*A*) | |
| | Warp coefficients (*W_{α}* for ∀*α*) | |
| Output | Transformed points ( ${X}_{o} = {\left\{{x}_{oj}\right\}}_{j = 1}^{M}$) | |
| 1 | Load 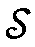 , *t, A, W_{α}* for ∀*α*, and *C* onto a shared memory space | |
| 2 | Parallel computation of eq. (1) on multiple input points simultaneously | |
| | | #pragma omp parallel for |
| | | for each point *xᵢⱼ:* |
| | | ${X}_{o} \left[j\right] = {x}_{ij} A + t + {\sum }_{α} U \left({x}_{ij}, {s}_{α}\right) {W}_{α}$ |

In addition to executing eq. (1) in parallel on multiple input points, $U \left(x, {s}_{α}\right) = {\left[U\left(x, {s}_{α}, {c}_{i}\right)\right]}_{i = 1}^{N}$ (and/or ${\left[U\left(\left‖x-{c}_{i}\right‖, {s}_{α}\right)\right]}_{i = 1}^{N}$), which is a vector with scalar-valued entries *U*(*x, s_{α}, cᵢ*) for each point *x* and each control point *cᵢ*, each scalar-valued entry *U*(*x, s_{α}, cᵢ*) can, according to the inventive technique, be computed in parallel since each *U*(*x, s_{α}, cᵢ*) is independent. Alternatively or in addition, assuming that there are N computing units, it will require N sequential calculation to compute *̅U̅*̅(*x, s_{α}*) on a single computing unit, or each element *U(x, s_{α}, cᵢ*) of *̅U̅*̅(*x, s_{α}*) can be computed (in particular independently) on each machine. Only one computation needs to be done on each machine to get the whole vector *̅U̅*̅(*x, s_{α}*)*.*

Additionally, *U*(*x, s_{α}*) for each support (and/or width of support) *s_{α}* is independent from the other supports, such that a set of basis functions $U \left(x\right) = \left\{U\left(x, {s}_{α}\right)\right\} = \left\{{\left[U\left(\left‖x-{c}_{i}\right‖, {s}_{α}\right)\right]}_{i = 1}^{N}\right\}$ can be computed simultaneously for all supports *s_{α}* in 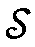 and all control points *cᵢ* in C independently as shown in the exemplary Algorithm II.

### Algorithm II: Multiprocessing of basis functions

| | | |
|---|---|---|
| Input | Input points (*x*) | |
| | Control points ( $C = {\left\{{c}_{j}\right\}}_{j = 1}^{N}$) | |
| | A set of CSRBF support ( 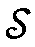 = {*s_{α}*}) | |
| Output | A set of basis functions ***U*** (*x*) | |
| 1 | Create basis function ***U*** (*x*) as a tensor of size \| 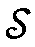 \| by *N* and initialize to zero | |
| 2 | Parallel calculation of basis function per *s_{α}* per *cⱼ* | |
| | # pragma omp parallel for collapse (2) | |
| | for each support *s_{α}* | |
| 3 | | for *cⱼ* in *C* |
| 4 | | $U \left[α, j\right] = U \left(x, {s}_{α}, {c}_{j}\right)$ |

Moreover, for compactly-supported RBF (CSRBF), *U*(*x, s_{α}*) is sparse since *U*(*x, s_{α}, cⱼ*) evaluates to zero if *x* is outside of the support of a control point *cⱼ*. Besides benefits in terms of speed and memory usage from sparse matrix operations (in particular compared to dense matrix operations), not all *U*(*x, s_{α}, cⱼ*) needs to be evaluated.

The exemplary Algorithm III incorporates a spatial tree/graph to query control points whose support cover *x* and evaluates *U*(*x, s_{α}, cⱼ*) only on these selected control points simultaneously.

### Algorithm III: Multiprocessing of compactly-supported basis functions

| | |
|---|---|
| Input | Input points (*x*) |
| | Control points ( $C = {\left\{{c}_{j}\right\}}_{j = 1}^{N}$) |
| | A set of CSRBF support ( 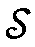 = {*s_{α}*}) |
| Output | A set of basis functions ***U*** (*x*) |
| 1 | Create basis function ***U*** (*x*) as a tensor of size \| 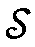 \| by *N* and initialize to zero |
| 2 | Create a spatial tree/graph of control points tree = Tree(*C*) |
| 3 | Query control points whose support covers *x* for all supports in 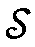 |

| | | |
|---|---|---|
| | | indices[α] = tree(*x*, radius = *s_{α}*) |
| 4 | Parallel calculation of basis function per *s_{α}* per active control point # pragma omp parallel for collapse (2) for each support *s_{α}* | |
| 5 | | for *i* in indices[α] |
| 6 | | $U \left[α, i\right] = U \left(x, {s}_{α}, {c}_{i}\right)$ |

It is noted that *U(x, s_{α}, cⱼ*) for each input point *x,* each control point *cⱼ*, and each (in particular fixed) support *s_{α}* is fixed and/or constant regardless of optimization iterations, multiresolution levels, or changes in the values of deformation parameters. Thus, *U(x, s_{α}, cⱼ*), ∀*x, s_{α}, cⱼ* can be computed once and stored to be used later in the subsequent optimization iteration and the next multiresolution level.

It is noted that in the Algorithm III, ***U***(*x*) denotes a tensor of size | 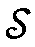 | by *N* where *N* is the number of control points, and ***U***[*α, i*]= *U(x, s_{α}, cᵢ*) is a scalar-valued entry.

In some embodiments, supports are fixed and set by users before execution of the inventive deformable registration technique. According to other examples, adaptive and/or automatically-determined supports may be possible as well.

Combing Algorithm I and Algorithm III may lead to Algorithm IV. Algorithm III on lines #5-6 can be replaced by Algorithm II lines# 3-4 if the RBF is globally supported, or by a read operation if *U*(*x, s_{α}, cⱼ*), ∀*x, s_{α}, cⱼ* are precomputed and stored elsewhere.

### Algorithm IV: Spline-based transformation of multiple input points simultaneously

| | | |
|---|---|---|
| Input | Input points ( ${X}_{i} = {\left\{{x}_{ij}\right\}}_{j = 1}^{M}$) | |
| | Control points ( $C = {\left\{{c}_{j}\right\}}_{i = 1}^{N}$) | |
| | CSRBF support ( 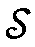 = {*s_{α}*}) | |
| | Offset (*t*) | |
| | Affine matrix (*A*) | |
| | Warp coefficients (*W_{α}* for ∀*α*) | |
| Output | Transformed points ( ${X}_{o} = {\left\{{x}_{oj}\right\}}_{j = 1}^{M}$) | |
| 1 | Load 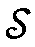 *, t, A, W_{α}* for ∀*α*, and *C* onto a shared memory space | |
| 2 | Parallel computation of eq. (1) on multiple input points simultaneously | |
| | | #pragma omp parallel for |
| 3 | | for each point *xᵢⱼ*: |
| 4 | | compute ***U*** (*xᵢⱼ*) using Algorithm III or II or read pre-computed *U*(*xᵢⱼ*) |
| 5 | | ${X}_{o} \left[j\right] = {x}_{ij} A + t + {\sum }_{α} U \left[α\right] \left({x}_{ij}\right) {W}_{α}$ |

It is noted that in Algorithm IV, ***U***[*α*] is a vector of ***U***[*α, i]* for*i* = 1,.., *N.* In other words, ***U***[*α, i]* = *U*(*x, s_{α}, cᵢ*) in Algorithm III is a scalar, while ***U***[*α*] *= U(x, s_{α}*) in Algorithm IV is *a N* × *d* vector.

Similar to other registration methods, the transformation in eq. (1) is estimated by optimizing an objective function involving data terms and regularization terms. To further speed up optimization and reduce memory usage, the inventive technique uses a derivative-based optimizer based on analytical derivatives (e.g., in comparison to a gradient-free optimization method or a gradient-based optimization method using automatic gradient calculation). In particular first-order derivatives are used for the optimization.

Second-order derivative-based optimization (e.g., involving Hessians) is also possible but computational complexity could outweigh optimization speed and stability.

Below, examples of the data terms and regularization terms are provided that can be used to optimize eq. (1) according to the inventive technique. Other data term and regularization not described below can also be integrated in the inventive technique. In what follows, the dissimilarity measures used as data terms and penalties used to regularize the deformation (and/or used as physical constraints) are described. Their analytical derivatives with respect to parameters of eq. (1) are provided, including *A, t,* and *W_{α},* ∀*α.*

An active control point scheme is described that can help to further reduce memory requirements while maintaining or improving alignment accuracy.

Given a set *X*₀ of points from a moving image domain (and/or the second medical image) and a set *X*₁ of points from a fixed image domain (and/or the first medical image, e.g., acting as reference image), instead of measuring the distance between them using a discrete point distance function (such as the L^{p} norm) requiring known point correspondence, each point set may be modeled as a mixture, and the dissimilarity between the two mixtures after transformation may be measured using L², denoted as *MD*(·,·), as ${L}_{P} \left(\left.{X}_{0}, {X}_{1}\right|ψ,ψ′\right) = {λ}_{g} MD \left({X}_{0},ψ\left[{X}_{1}\right]\right) + {λ}_{g ′} MD \left(ψ′\left[{X}_{0}\right],{X}_{1}\right) ,$ (2) where *ψ* is a transformation mapping points in *X*₁ to *X*₀, and *ψ'* maps *X*₀ to *X*₁*.* E.g., *ψ* may be deformable registration function of eq. (1) and *ψ'* its inverse.

*MD*(·,·) denotes the L² distance between mixtures, which is described further below in the context of point cloud dissimilarity. Alternatively or in addition to the point could dissimilarity, image dissimilarity, such as mean squared error, $MSE \left({I}_{0},{I}_{1}\left|ψ,ψ′\right.\right) = \frac{{λ}_{ms}}{\left|Ω\right|} {\sum }_{x ∈ Ω} {\left({I}_{0}∘ψ\left(x\right)-{I}_{1}\left(x\right)\right)}^{2} + \frac{{λ}_{ms ′}}{\left|Ω′\right|} {\sum }_{y ∈ Ω ′} {\left({I}_{0}\left(y\right)-{I}_{1}∘ψ′\left(y\right)\right)}^{2} ,$ (3) between a moving image *I*₀ (e.g., the second medical image) and a fixed image *I*₁ (e.g., the first medical image) after transformation may be minimized. Further alternatively or in addition, gradient information [7] may be maximized, $\begin{matrix}GI \left({I}_{0},{I}_{1}\left|ψ,ψ′\right.\right) = \frac{{λ}_{GI}}{\left|Ω\right|} {\sum }_{x ∈ Ω} {\left(\frac{\left({∇}_{ψ \left(x\right)}\left({I}_{0}∘ψ\left(x\right)\right)\right) ⋅ \left({∇}_{x}{I}_{1}\right)}{\left‖{∇}_{ψ \left(x\right)}\left({I}_{0}∘ψ\left(x\right)\right)\right‖ \left‖{∇}_{x}{I}_{1}\right‖}\right)}^{2} min \left({\left‖{∇}_{ψ \left(x\right)}\left({I}_{0}∘ψ\left(x\right)\right)\right‖}_{2}, {\left‖{∇}_{x}{I}_{1}\right‖}_{2}\right) + \\ \frac{{λ}_{GI ′}}{\left|Ω′\right|} {\sum }_{y ∈ Ω ′} {\left(\frac{\left({∇}_{y}{I}_{0}\right) ⋅ \left({∇}_{ψ ′ \left(y\right)}\left({I}_{1}∘ψ′\left(y\right)\right)\right)}{\left‖{∇}_{ψ ′ \left(y\right)}\left({I}_{1}∘ψ′\left(y\right)\right)\right‖ \left‖{∇}_{y}{I}_{0}\right‖}\right)}^{2} min \left({\left‖{∇}_{ψ ′ \left(y\right)}\left({I}_{1}∘ψ′\left(y\right)\right)\right‖}_{2}, {\left‖{∇}_{y}{I}_{0}\right‖}_{2}\right)\end{matrix}$ (4)

It is noted that eq. (4) is a modification of the gradient information proposed in [7] to improve optimization stability.

Alternatively or in addition to image dissimilarity and point cloud dissimilarity, the inventive technique can incorporate a landmark matching constraint defined as, ${L}_{lmk} \left({L}_{0},{L}_{1}\left|ψ,ψ′\right.\right) = \frac{1}{{N}_{L}} {\sum }_{i = 1}^{{N}_{L}} {\left‖{l}_{0 , i}-ψ\left({l}_{1 , i}\right)\right‖}_{2}^{2} + \frac{1}{{N}_{L}} {\sum }_{i = 1}^{{N}_{L}} {\left‖{l}_{1 , i}-ψ′\left({l}_{0 , i}\right)\right‖}_{2 ′}^{2}$ (5) where ${L}_{0} = {\left\{{l}_{0 , i}\right\}}_{i = 1}^{{N}_{L}}$ and ${L}_{1} = {\left\{{l}_{1 , i}\right\}}_{i = 1}^{{N}_{L}}$ are sets of known landmark correspondences in the second medical image (and/or *I*₀) and in the first medical image (and/or *I*₁), respectively.

Alternatively or in addition, various penalties (and/or physical constraints) can be imposed to constrain the solution space, therefore improving well-posedness, optimization stability, and physically plausible deformation outcomes.

The penalties may include a sliding motion constraint computed per sliding structure S as, ${R}_{S} \left(ψ\right) = \frac{1}{\left|{X}_{S}\right|} {\sum }_{x ∈ {X}_{S}} \frac{\left|n\left(x\right){∇}_{x}ψ⋅u\left(x\right)\right|}{r {\left(x\right)}^{2} {\left‖u\left(x\right)\right‖}_{2}} ,$ (6) where *n(x)* denotes the surface normal at the surface point *x ∈ X_{S}* ⊂ *X*₁, *u(x)* = *ψ*(*x*) *- x* is the displacement at *x,* ∇*ₓψ* is the derivative of *ψ* with respect to *x,* and $r \left(x\right) = {min}_{y ∈ {X}_{0 \ S}} {\left‖ψ\left(x\right)-y\right‖}_{2}$ is the minimum distance between x and surface points y ∈ *X*_{0\}*_{S}.* The sliding motion constraint on *ψ'* can be derived in the similar manner. In Eq. (6), *X_{S}* is a set of surface points (and/or vectors) of organs capable of sliding against other organs.

Alternatively or in addition, rigid motion of bony structures (also: bone rigidity constraint) may be imposed through, ${R}_{R} \left(ψ\right) = {\left‖{∇}_{x}{ψ}^{T}{∇}_{x}ψ-I\right‖}_{F}^{2} + {\left‖{∇}_{x}ψ{∇}_{x}{ψ}^{T}-I\right‖}_{F}^{2}$ (7) for *ψ*, and a similar constraint can be derived for *ψ'*. To reduce computational expense, *R_{R}* can be defined using only one Frobenius norm term in eq. (4) (e.g., either the first term or last term).

Alternatively or in addition, tissue incompressibility may be imposed via, ${R}_{I} \left(ψ\right) = {\left(det\left({∇}_{x}ψ\right)-1\right)}^{2} .$ (8)

Alternatively or in addition, smoothness may be imposed via affinity or bending energy penalty as, ${R}_{A} \left(ψ\right) = {E}_{α} \left[tr\left({W}_{α}^{T}{K}_{α}{W}_{α}\right)\right] ,$ (9) where ${K}_{α} ∈ {ℝ}^{N \times N}$ is a matrix of basis functions of control points. *R_{I}* and *R_{A}* for *ψ*' can be derived in the similar manner.

*U* is generally computed between input points and control points. The matrix *K* in eq. (9) is computed essentially the same way by just replacing input points with control points. In other words, *K* is computed between control points ${K}_{α} \left({c}_{i}\right) = U \left({c}_{i}, {s}_{α}\right) = {\left[U\left(\left‖{c}_{i}-{c}_{j}\right‖, {s}_{α}\right)\right]}_{j = 1}^{N}$.

Alternatively or in addition, topology preservation may be imposed via an inverse consistency constraint defined as ${R}_{I} = {\left‖ψ′∘ψ\left(x\right)-x\right‖}_{2} + {\left‖ψ∘ψ′\left(y\right)-y\right‖}_{2} ,$ (10) where *x ∈ X₁* and *y ∈ X₀*. Similar to *R_{R}*, to reduce computation expense, *Rₗ* can be implemented using a single term in eq. (7)(e.g., either first term or last term).

The total objective function may thereof read:${argmin}_{Ψ , Ψ '} \left\{\begin{matrix}{L}_{p} \left({X}_{0},{X}_{1}\left|Ψ,Ψ'\right.\right) + MSE \left({I}_{0},{I}_{1}\left|Ψ,Ψ'\right.\right) + GI \left({I}_{0},{I}_{1}\left|Ψ,Ψ'\right.\right) + {λ}_{Lm} {L}_{lmk} \left({L}_{0},{L}_{1}\left|Ψ,Ψ'\right.\right) \\ + {λ}_{S} {R}_{S} \left(Ψ\right) + {λ}_{S '} {R}_{S} \left(Ψ'\right) + {λ}_{R} {R}_{R} \left(Ψ\right) + {λ}_{R '} {R}_{R} \left(Ψ'\right) + {λ}_{A} {R}_{A} \left(Ψ\right) + {λ}_{A '} {R}_{A} \left(Ψ'\right) \\ + {λ}_{l} {R}_{l} \left(Ψ\right) + {λ}_{l '} {R}_{l} \left(Ψ'\right)\end{matrix}\right\} .$ (11)

As mentioned previously, other data terms measuring image dissimilarity and/or other regularizations can be easily integrated into the inventive technique. In what follows, analytical derivatives of each loss and penalty term with respect to parameters of transformations (and/or deformation functions) of the form defined in eq.(1) including *W_{α}* ∀*α, A,* and *t* are provided.

### Point cloud dissimilarity

Alternatively to discrete dissimilarity, a discrete point set $X = {\left\{{x}_{i}\right\}}_{i = 1}^{M}$ may be represented as a mixture of continuous distribution, such as a Gaussian mixture $φ \left(\left.x\right|θ\right) = {\sum }_{i = 1}^{N} {w}_{i} ϕ \left(\left.x\right|{μ}_{i},{Σ}_{i}\right) ,$ where *wᵢ* is a density weight that can be used to weigh down points with high uncertainty, and *ϕ(x*|*µᵢ*, *Σᵢ*) is a Gaussian density with mean *µᵢ* and covariance Σ*ᵢ*, $ϕ \left(\left.x\right|μ,Σ\right) = \frac{exp \left[-0.5{\left(x-μ\right)}^{T}{Σ}^{- 1}\left(x-μ\right)\right]}{\sqrt{{\left(2π\right)}^{d} \left|det\left(Σ\right)\right|}} .$

The dissimilarity between the mixture of a moving point set *X*₀ (e.g., representing the second medical image) and a fixed point set *X*₁ (e.g., representing the first medical image) after transformation *ψ* can be computed as the L² distance, $MD \left({X}_{0},ψ\left[{X}_{1}\right]\right) = \int {\left[φ\left(\left.x\right|θ 0\right)-φ\left(\left.x\right|ψ\left(θ 1\right)\right)\right]}^{2} dx ,$ where *^{k}θ* is a set of mixture parameters including { *^{k}_{Wi}*, *^{k}µᵢ, ^{k}Σᵢ*}.Focusing on the derivatives of *MD*(*X*₀, *ψ*[*X*₁]), since *MD*(*ψ'*[*X*₀], *X*₁) can be derived similarly, gives: $MD \left({X}_{0},ψ\left[{X}_{1}\right]\right) = \int φ {\left(\left.x\right|θ 0\right)}^{2} dx - 2 \int φ \left(\left.x\right|θ 0\right) φ \left(\left.x\right|ψ\left(θ 1\right)\right) dx + \int φ {\left(\left.x\right|ψ\left(θ 1\right)\right)}^{2} dx .$ (12)

The first term does not depend on transformation parameters (i.e., its derivative with respect to the transformation parameters is zeros). Thus, only the last two terms contribute to the derivative of (12). For Gaussian mixtures, the Gaussian mixture identity, $\int φ \left(\left.x\right|θ 1\right) φ \left(\left.x\right|θ 2\right) dx = {\sum }_{i = 1}^{m} {\sum }_{j = 1}^{n} w i 1 w j 2 ϕ \left(\left.0\right|μ i 1-μ j 2, Σ i 1+Σ j 2\right) ,$ can be used.

The second term in eq (12) can be written as $\begin{matrix}\int φ \left(\left.x\right|θ 0\right) φ \left(\left.x\right|ψ\left(θ 1\right)\right) dx = {\sum }_{i = 1}^{m} {\sum }_{j = 1}^{n} w j 0 w i 1 ϕ \left(\left.0\right|μ j 0-ψ\left(μ i 1\right),Σ j 0+Σ i 1\right) \\ = {\sum }_{i = 1}^{m} {\sum }_{j = 1}^{n} w j 0 w i 1 \frac{exp \left[-\left(μ j 0-ψ\left(μ j 1\right)\right){\left(Σ j 0+Σ i 1\right)}^{- 1}{\left(μ j 0-ψ\left(μ j 1\right)\right)}^{T}\right]}{2 \sqrt{{\left(2π\right)}^{d} \left|det\left(Σ j 0+Σ i 1\right)\right|}} \\ = {\sum }_{i = 1}^{m} {\sum }_{j = 1}^{n} w j 0 w i 1 exp \left[-\left(μ j 0-ψ\left(μ i 1\right)\right){\left(Σ j 0+Σ i 1\right)}^{- 1}{\left(μ j 0-ψ\left(μ j 1\right)\right)}^{T}\right]\end{matrix}$ (13) where ⁰*µⱼ* denotes a j-th point in the set *X*₀ and ¹*µᵢ* denotes an *i*-th point in the set *X*₁, and it is assumed that the normalization term $2 \sqrt{{\left(2π\right)}^{d} \left|det\left(Σ j 0+Σ i 1\right)\right|}$ is absorbed into the variance.

Similarly, the last term in eq. (12) becomes, $\begin{matrix}\int {φ}^{2} \left(\left.x\right|ψ\left(θ 1\right)\right) dx = {\sum }_{i = 1}^{m} {\sum }_{j = 1}^{m} w i 1 w j 1 ϕ \left(\left.0\right|ψ\left(μ i 1\right)-ψ\left(μ j 1\right),Σ i 1+Σ j 1\right) \\ = {\sum }_{i = 1}^{m} {\sum }_{j = 1}^{m} w i 1 w j 1 \frac{exp \left[-\left(ψ\left(μ i 1\right)-ψ\left(μ j 1\right)\right){\left(Σ i 1+Σ j 1\right)}^{- 1}{\left(ψ\left(μ i 1\right)-ψ\left(μ j 1\right)\right)}^{T}\right]}{2 \sqrt{{\left(2π\right)}^{d} \left|det\left(Σ i 1+Σ j 1\right)\right|}}\end{matrix}$ (14) $= {\sum }_{i = 1}^{m} {\sum }_{j = 1}^{m} w i 1 w j 1 exp \left[-\left(ψ\left(μ i 1\right)-ψ\left(μ j 1\right)\right){\left(Σ i 1+Σ j 1\right)}^{- 1}{\left(ψ\left(μ i 1\right)-ψ\left(μ j 1\right)\right)}^{T}\right]$

The derivative of eq. (13) with respect to *W_{α}* is ${\partial }_{{W}_{α}} \int φ \left(\left.x\right|θ 0\right) φ \left(\left.x\right|ψ\left(θ 1\right)\right) dx = {\sum }_{i = 1}^{m} {\sum }_{j = 1}^{n} 2 {zU}_{α}^{T} \left(μ j 0-ψ\left(μ i 1\right)\right) {\left(Σ j 0+Σ i 1\right)}^{- 1} ,$ (15) where $z = w j 0 w i 1 exp \left[-\left(μ j 0-ψ\left(μ i 1\right)\right){\left(Σ j 0+Σ i 1\right)}^{- 1}{\left(μ j 0-ψ\left(μ i 1\right)\right)}^{T}\right] .$

The derivative of eq. (13) with respect to *A* is ${\partial }_{A} \int φ \left(\left.x\right|θ 0\right) φ \left(\left.x\right|ψ\left(θ 1\right)\right) dx = 2 z {\left(μ i 1\right)}^{T} \left(μ j 0-ψ\left(μ i 1\right)\right) {\left(Σ j 0+Σ i 1\right)}^{- 1} .$ (16)

The derivative of eq. (13) with respect to t is ${\partial }_{t} \int φ \left(\left.x\right|θ 0\right) φ \left(\left.x\right|ψ\left(θ 1\right)\right) dx = 2 z \left(μ j 0-ψ\left(μ i 1\right)\right) {\left(Σ j 0+Σ i 1\right)}^{- 1}$ (17)

Likewise, the derivative of eq. (14) with respect to *W_{α}* is, ${\partial }_{{W}_{α}} \int φ {\left(\left.x\right|ψ\left(θ 1\right)\right)}^{2} dx = {\sum }_{i = 1}^{m} {\sum }_{j = 1}^{m} - 2 z {\left({U}_{α}\left(μ i 1\right)-{U}_{α}\left(μ j 1\right)\right)}^{T} \left(ψ\left(μ i 1\right)-ψ\left(μ j 1\right)\right) {\left(Σ i 1+Σ j 1\right)}^{- 1} ,$ (18) where *U_{α}*(·) = *U*(·, *s_{α}*), z = ¹*wᵢ* ¹*wⱼ* exp[-(*ψ*( ¹*µᵢ*)-*ψ*( ¹*µⱼ*))( 1Σ*ⱼ* + ¹*Σⱼ*)⁻¹(*ψ*( ¹*µᵢ*)-*ψ*( ¹*µⱼ*))*^{T}*].

The derivative of eq. (14) with respect to *A* is ${\partial }_{A} \int φ {\left(\left.x\right|ψ\left(θ 1\right)\right)}^{2} dx = {\sum }_{i = 1}^{m} {\sum }_{j = 1}^{m} - 2 z {\left(μ i 1-μ j 1\right)}^{T} \left(ψ\left(μ j 1\right)-ψ\left(μ j 1\right)\right) {\left(Σ i 1+Σ j 1\right)}^{- 1} .$ (19)

We note that ∂ₜ *∫ φ* (*x*|*ψ*( ¹*θ*))² *dx =* 0.

### Mean squared error

The mean squared error (MSE) in eq. (3) measures dissimilarity between *I*₁ (and/or the first medical image) and *I*₀ (and/or the second medical image) after transformation, *I*₀ o *ψ*, and between *I*₀ and *I*₀ after transformation, *I*₁ ∘ *ψ'*, assuming that *I*₀ and *I*₁ are acquired from the same imaging modality. Since the derivation of the derivative of MSE between *I*₀ o *ψ*(*x*) and *I*₁ is similar to the derivation of the MSE derivative between *I*₁ o *ψ'* and *I*₀, focusing only on the derivative of MSE between *I*₀ o *ψ*(*x*) and *I*₁ (i.e., the first term in eq. (3)) leads to the following result. The derivative with respect to the *mn-*th element of *W_{α}* is, ${\partial }_{{\left[{W}_{α}\right]}_{mn}} \frac{1}{\left|Ω\right|} {\sum }_{x ∈ Ω} {\left({I}_{0}∘ψ\left(x\right)-{I}_{1}\left(x\right)\right)}^{2}$ $= \frac{1}{\left|Ω\right|} {\sum }_{x ∈ Ω} 2 \left({I}_{0}∘ψ\left(x\right)-{I}_{1}\right) {U}_{α} {J}^{mn} {\left[{∇}_{ψ \left(x\right)}\left({I}_{0}∘ψ\left(x\right)\right)\right]}^{T} .$

Likewise, the derivative with respect to the *mn*-th element of *A* is, ${\partial }_{{\left[A\right]}_{mn}} \frac{1}{\left|Ω\right|} {\sum }_{x ∈ Ω} {\left({I}_{0}∘ψ\left(x\right)-{I}_{1}\left(x\right)\right)}^{2} = \frac{1}{\left|Ω\right|} {\sum }_{x ∈ Ω} 2 \left({I}_{0}∘ψ\left(x\right)-{I}_{1}\right) {xJ}^{mn} {\left[{∇}_{ψ \left(x\right)}\left({I}_{0}∘ψ\left(x\right)\right)\right]}^{T} ,$ and the *m*-th element of *t* is, ${\partial }_{{\left[t\right]}_{m}} \frac{1}{\left|Ω\right|} {\sum }_{x ∈ Ω} {\left({I}_{0}∘ψ\left(x\right)-{I}_{1}\left(x\right)\right)}^{2} = \frac{1}{\left|Ω\right|} {\sum }_{x ∈ Ω} 2 \left({I}_{0}∘ψ\left(x\right)-{I}_{1}\right) {J}^{m} {\left[{∇}_{ψ \left(x\right)}\left({I}_{0}∘ψ\left(x\right)\right)\right]}^{T} .$ Ω herein is the image area, image grid, and/or image domain, and/or Ω can be the same as *Xᵢ.*

### Gradient information

Similar to eq. (3), gradient information (GI) in eq. (4) comprises two terms. Unlike eq. (3), GI measures similarity between *I*₁ (and/or the first medical image) and *I*₀ (and/or the second medical image) after transformation, *I*₀ o *ψ*, and between *I*₀ and *I*₀ after transformation, *I*₁ ∘ *ψ'*, without assuming that *I*₀ and *I*₀ are acquired from the same imaging modality. Since the derivatives of the first term and the second term can be derived in a similar manner, only the derivatives of the first term are provided. $GI = {\sum }_{x ∈ Ω} {\left(\frac{\left({∇}_{ψ \left(x\right)}\left({I}_{0}∘ψ\left(x\right)\right)\right) ⋅ \left({∇}_{x}{I}_{1}\right)}{\left‖{∇}_{ψ \left(x\right)}\left({I}_{0}∘ψ\left(x\right)\right)\right‖ \left‖{∇}_{x}{I}_{1}\right‖}\right)}^{2} min \left({\left‖{∇}_{ψ \left(x\right)}\left({I}_{0}∘ψ\left(x\right)\right)\right‖}_{2}, {\left‖{∇}_{x}{I}_{1}\right‖}_{2}\right) = {\sum }_{x ∈ Ω} {A}^{2} B$

Let $A = \left(\frac{\left({∇}_{ψ \left(x\right)}\left({I}_{0}∘ψ\left(x\right)\right)\right) ⋅ \left({∇}_{x}{I}_{1}\right)}{\left‖{∇}_{ψ \left(x\right)}\left({I}_{0}∘ψ\left(x\right)\right)\right‖ \left‖{∇}_{x}{I}_{1}\right‖}\right) and B = min \left({\left‖{∇}_{ψ \left(x\right)}\left({I}_{0}∘ψ\left(x\right)\right)\right‖}_{2}, {\left‖{∇}_{x}{I}_{1}\right‖}_{2}\right) .$

The derivative of GI with respect to the *mn-*th element of *W_{α}* is ${\partial }_{{\left[{W}_{α}\right]}_{mn}} GI = {\sum }_{x ∈ Ω} \left\{{\partial }_{{\left[{W}_{α}\right]}_{mn}}{A}^{2}B+{A}^{2}{\partial }_{{\left[{W}_{α}\right]}_{mn}}B\right.$ (20) where $\begin{matrix}{\partial }_{{\left[{W}_{α}\right]}_{mn}} {A}^{2} \\ = 2 A \left\{\begin{matrix}\frac{{U}_{α} {J}^{mn} {\left[{H}_{ψ}\left({I}_{0}∘ψ\left(x\right)\right)\right]}^{T} {\left({∇}_{x}{I}_{1}\right)}^{T}}{\left‖{∇}_{ψ \left(x\right)}\left({I}_{0}∘ψ\left(x\right)\right)\right‖ \left‖{∇}_{x}{I}_{1}\right‖} \\ - \frac{\left\{\left({∇}_{ψ \left(x\right)}\left({I}_{0}∘ψ\left(x\right)\right)\right)⋅\left({∇}_{x}{I}_{1}\right)\right\} {U}_{α} {J}^{mn} {\left[{H}_{ψ \left(x\right)}\left({I}_{0}∘ψ\left(x\right)\right)\right]}^{T} {\left[{∇}_{ψ \left(x\right)}\left({I}_{0}∘ψ\left(x\right)\right)\right]}^{T}}{{\left‖{∇}_{ψ \left(x\right)}\left({I}_{0}∘ψ\left(x\right)\right)\right‖}^{3} \left‖{∇}_{x}{I}_{1}\right‖}\end{matrix}\right\}\end{matrix}$ and ${\partial }_{{\left[{W}_{α}\right]}_{mn}} B = \left\{\begin{matrix}0 if {\left‖{∇}_{ψ \left(x\right)}\left({I}_{0}∘ψ\left(x\right)\right)\right‖}_{2} > {\left‖{∇}_{x}{I}_{1}\right‖}_{2} \\ \frac{{U}_{α} {J}^{mn} {\left[{H}_{ψ \left(x\right)}\left({I}_{0}∘ψ\left(x\right)\right)\right]}^{T} {\left[{∇}_{ψ \left(x\right)}\left({I}_{0}∘ψ\left(x\right)\right)\right]}^{T}}{{\left‖{∇}_{ψ \left(x\right)}\left({I}_{0}∘ψ\left(x\right)\right)\right‖}_{2}} , otherwise\end{matrix}\right. .$

Similarly, the derivative ∂_{[*A*]*mn*} GI follows eq. (20) with ${\partial }_{{\left[{W}_{α}\right]}_{mn}} {A}^{2} = 2 A \left(\begin{matrix}\frac{{xJ}^{mn} {\left[{H}_{ψ \left(x\right)}\left({I}_{0}∘ψ\left(x\right)\right)\right]}^{T} {\left({∇}_{x}{I}_{1}\right)}^{T}}{\left‖{∇}_{ψ \left(x\right)}\left({I}_{0}∘ψ\left(x\right)\right)\right‖ \left‖{∇}_{x}{I}_{1}\right‖} - \\ \frac{\left\{\left({∇}_{ψ \left(x\right)}\left({I}_{0}∘ψ\left(x\right)\right)\right)\left({∇}_{x}{I}_{1}\right)\right\} \left({xJ}^{mn}{\left[{H}_{ψ \left(x\right)}\left({I}_{0}∘ψ\left(x\right)\right)\right]}^{T}{\left[{∇}_{ψ \left(x\right)}\left({I}_{0}∘ψ\left(x\right)\right)\right]}^{T}\right)}{{\left‖{∇}_{ψ \left(x\right)}\left({I}_{0}∘ψ\left(x\right)\right)\right‖}^{3} \left‖{∇}_{x}{I}_{1}\right‖}\end{matrix}\right)$ and ${\partial }_{{\left[{W}_{α}\right]}_{mn}} B = \left\{\begin{matrix}0 if {\left‖{∇}_{ψ \left(x\right)}\left({I}_{0}∘ψ\left(x\right)\right)\right‖}_{2} > {\left‖{∇}_{x}{I}_{1}\right‖}_{2} \\ \frac{{xJ}^{mn} {\left[{H}_{ψ \left(x\right)}\left({I}_{0}∘ψ\left(x\right)\right)\right]}^{T} {\left({∇}_{ψ \left(x\right)}\left({I}_{0}∘ψ\left(x\right)\right)\right)}^{T}}{{\left‖{∇}_{ψ \left(x\right)}\left({I}_{0}∘ψ\left(x\right)\right)\right‖}_{2}} , otherwise\end{matrix}\right. .$

Finally, the derivative ∂_{[*t*]*m*} GI, likewise, follows eq. (20) with ${\partial }_{{\left[t\right]}_{m}} {A}^{2} = 2 A \left\{\begin{matrix}\frac{{J}^{m} {\left[{H}_{ψ \left(x\right)}\left({I}_{0}∘ψ\left(x\right)\right)\right]}^{T} {\left[\left({∇}_{x}{I}_{1}\right)\right]}^{T}}{\left‖{∇}_{ψ \left(x\right)}\left({I}_{0}∘ψ\left(x\right)\right)\right‖ \left‖{∇}_{x}{I}_{1}\right‖} - \\ \frac{\left\{\left({∇}_{ψ \left(x\right)}\left({I}_{0}∘ψ\left(x\right)\right)\right)⋅{\left[\left({∇}_{x}{I}_{1}\right)\right]}^{T}\right\} {J}^{m} {\left[{H}_{ψ \left(x\right)}\left({I}_{0}∘ψ\left(x\right)\right)\right]}^{T} {\left[{∇}_{ψ \left(x\right)}\left({I}_{0}∘ψ\left(x\right)\right)\right]}^{T}}{{\left‖{∇}_{ψ \left(x\right)}\left({I}_{0}∘ψ\left(x\right)\right)\right‖}^{3} \left‖{∇}_{x}{I}_{1}\right‖}\end{matrix}\right\}$ and ${\partial }_{{\left[t\right]}_{m}} B = \left\{\begin{matrix}0 if {\left‖{∇}_{ψ \left(x\right)}\left({I}_{0}∘ψ\left(x\right)\right)\right‖}_{2} > {\left‖{∇}_{x}{I}_{1}\right‖}_{2} \\ \frac{{J}^{m} {\left[{H}_{ψ \left(x\right)}\left({I}_{0}∘ψ\left(x\right)\right)\right]}^{T} {\left[{∇}_{ψ \left(x\right)}\left({I}_{0}∘ψ\left(x\right)\right)\right]}^{T}}{{\left‖{∇}_{ψ \left(x\right)}\left({I}_{0}∘ψ\left(x\right)\right)\right‖}_{2}} , otherwise\end{matrix}\right. .$

### Landmark matching constraint

The landmark matching constraint defined in eq. (5) comprises two terms imposing geometric accuracy of the estimated transformations *ψ* and *ψ'*, respectively. Since the derivative of each term with respect to parameters of *ψ* and *ψ'* can be derived in a similar manner, only the derivative with respect to *ψ* is provided. The derivative of eq. (5) with respect to *W_{α}* in eq. (1) is, $\frac{1}{{N}_{L}} {\sum }_{i}^{{N}_{L}} {\partial }_{{W}_{α}} {\left‖{l}_{0 , i}-ψ\left({l}_{1 , i}\right)\right‖}_{2}^{2} = \frac{- 2}{{N}_{L}} {\sum }_{i}^{{N}_{L}} {U}_{α}^{T} \left({l}_{1 , i}\right) \left({l}_{0 , i}-ψ\left({l}_{1 , i}\right)\right) .$

Likewise, the derivative with respect to *A* is, $\frac{1}{{N}_{L}} {\sum }_{i}^{{N}_{L}} {\partial }_{A} {\left‖{l}_{0 , i}-ψ\left({l}_{1 , i}\right)\right‖}_{2}^{2} = \frac{- 2}{{N}_{L}} {\sum }_{i}^{{N}_{L}} {l}_{1 , i}^{T} \left({l}_{0 , i}-ψ\left({l}_{1 , i}\right)\right) ,$ and with respect to t is, $\frac{1}{{N}_{L}} {\sum }_{i}^{{N}_{L}} {\partial }_{A} {\left‖{l}_{0 , i}-ψ\left({l}_{1 , i}\right)\right‖}_{2}^{2} = \frac{- 2}{{N}_{L}} {\sum }_{i}^{{N}_{L}} \left({l}_{0 , i}-ψ\left({l}_{1 , i}\right)\right) .$

### Sliding motion constraint

The sliding motion constraint defined in eq. (6) may be used to model organ interfaces that slide against each other, such as the lung sliding against the ribs and/or the liver sliding against the spine and the nearby soft-tissue organs (e.g., kidneys). In the following, only the derivative of eq. (6) with respect to the parameters of *ψ* is derived since the derivative of the sliding motion constraint with respect to the parameters of *ψ'* can be derived in a similar manner. The derivative of eq. (6) with respect to *W_{α}* in eq. (1) is, ${\partial }_{{W}_{α}} {R}_{S} = \frac{1}{\left|{X}_{S}\right|} \sum \left\{\begin{matrix}\frac{n {∇}_{x} {ψu}^{T} \left({∇}_{x}{U}_{α}{n}^{T}u+{U}_{α}^{T}n{∇}_{x}ψ\right)}{{r}^{2} {\left‖u\right‖}_{2} \left|n{∇}_{x}{ψu}^{T}\right|} - \\ \frac{\left|n{∇}_{x}ψ⋅u\right| \left[{\left‖u\right‖}_{2}{min}_{y}\left[2{U}_{α}^{T}\left(ψ\left(x\right)-y\right)\right]+{r}^{2}\frac{{U}_{α}^{T} u}{{\left‖u\left(x\right)\right‖}_{2}}\right]}{{\left({r}^{2}{\left‖u\right‖}_{2}\right)}^{2}}\end{matrix}\right\} ,$ (21

Where a displacement *u(x)* at a point *x* is denoted as *u* and a normal vector *n*(*x*) of a structure located at the point *x* is denoted as *n,* for brevity. Additionally, for the sake of brevity *U*(*x, s_{α}*) is denoted as *U_{α}.*

∇*ₓU_{α}* is the derivative of the basis function *U_{α}* with respect to *x.*

Algorithm II and III can be used to speed up calculation of *U_{α}* and ∇*ₓU_{α}* in eq. (21). Also, like U*_{α},* ∇*ₓU_{α}* are constant and can be pre-computed once, stored, and used in subsequent optimization. Likewise, the derivative of eq. (6) with respect to *A* is, ${\partial }_{A} {R}_{S} = \frac{1}{\left|{X}_{S}\right|} \sum \left\{\frac{n {∇}_{x} {ψu}^{T} \left({n}^{T}u+{x}^{T}n{∇}_{x}ψ\right)}{{r}^{2} {\left‖u\right‖}_{2} \left|n{∇}_{x}{ψu}^{T}\right|}-\frac{\left|n{∇}_{x}ψ⋅u\right| \left[{\left‖u\right‖}_{2}{min}_{y}\left[2{x}^{T}\left(ψ\left(x\right)-y\right)\right]+{r}^{2}\frac{{x}^{T} u}{{\left‖u\right‖}_{2}}\right]}{{\left({r}^{2}{\left‖u\right‖}_{2}\right)}^{2}}\right\} ,$ and the derivative with respect to *t* is ${\partial }_{t} {R}_{S} = \frac{1}{\left|{X}_{S}\right|} \sum \left\{\frac{n {∇}_{x} ψ \left({u}^{T}\left(n{∇}_{x}ψ\right)\right)}{{r}^{2} {\left‖u\right‖}_{2} \left|n{∇}_{x}{ψu}^{T}\right|}-\frac{\left|n{∇}_{x}ψ⋅u\right| \left[{\left‖u\right‖}_{2}{min}_{y}2\left(ψ\left(x\right)-y\right)+{r}^{2}\frac{u}{{\left‖u\right‖}_{2}}\right]}{{\left({r}^{2}{\left‖u\right‖}_{2}\right)}^{2}}\right\} .$

### Bone rigidity constraint

As shown in eq. (7), rigid motion of bony structures may be imposed through the orthogonal property of a rotation matrix. In other words, for motion at *x* to be rigid, the spatial derivative of the deformation function (also: transformation) in eq. (1) must be rotation. In what follows, the derivative of ${\left‖{∇}_{x}{ψ}^{T}{∇}_{x}ψ-I\right‖}_{F}^{2}$ in eq. (7) with respect to parameters of eq. (1) is derived. The derivative of ${\left‖{∇}_{x}ψ{∇}_{x}{ψ}^{T}-I\right‖}_{F}^{2}$ as well as the derivative of the constraint with respect to *ψ'* can be derived similarly. The derivative of ${\left‖{∇}_{x}{ψ}^{T}{∇}_{x}ψ-I\right‖}_{F}^{2}$ in eq. (7) with respect to the *mn-th* element of *W_{α}* is, ${\partial }_{{\left[{W}_{α}\right]}_{mn}} {\left‖{∇}_{x}{ψ}^{T}{∇}_{x}ψ-I\right‖}_{F}^{2} = {\sum }_{i = 1}^{d} {\sum }_{j = 1}^{d} 2 {\left({∇}_{x}{ψ}^{T}{∇}_{x}ψ-I\right)}_{ij} {\left({J}^{nm}{∇}_{x}{U}_{α}{∇}_{x}ψ+{\left({J}^{nm}{∇}_{x}{U}_{α}{∇}_{x}ψ\right)}^{T}\right)}_{ij}$ where *J^{mn}* is a matrix of the same dimension as *W* with all zeros except the *mn-th* element whose value is 1. Similarly, *J^{nm}* is a matrix of the same dimension as *W^{T}* with only the *nm*-th element having value 1. ∇*ₓU_{α}* is the derivative of the basis function *U_{α}* with respect to *x,* and similar to *U_{α},* its calculation can be facilitated by a spatial tree/graph and can be parallelized per support and per point (see Algorithms II and III).

The derivative of ${\left‖{∇}_{x}{ψ}^{T}{∇}_{x}ψ-I\right‖}_{F}^{2}$ with respect to the *mn-*th element of *A* is, ${\partial }_{{\left[A\right]}_{mn}} {\left‖{∇}_{x}{ψ}^{T}{∇}_{x}ψ-I\right‖}_{F}^{2} = {\sum }_{i = 1}^{d} {\sum }_{j = 1}^{d} 2 {\left({∇}_{x}{ψ}^{T}{∇}_{x}ψ-I\right)}_{ij} {\left({J}^{nm}{∇}_{x}ψ+{\left({J}^{nm}{∇}_{x}ψ\right)}^{T}\right)}_{ij} .$

It is noted that the penalty (and/or the bone rigidity constraint) does not depend on *t*, so its derivative with respect to *t* is a zero vector.

### Tissue incompressibility constraint

Besides eq. (8), the tissue incompressibility constraint can be defined as (log(det∇*ₓψ*))))². Although this form supports symmetric penalization of compression and expansion, it results in unstable optimization when det(∇*ₓψ*)) approaches zero and when ∇ₓ*ψ* is degenerate and/or singular since its derivative involves the inverse of ∇*ₓψ*).

Similar to eq. (7), eq. (8) does not depend on *t.* Thus, the derivative of eq. (8) with respect to *t* is a zero vector.

The derivative of eq. (8) with respect to the *mn-th* element of *W_{α}* is ${\partial }_{{\left[{W}_{α}\right]}_{mn}} {\left(det\left({∇}_{x}ψ\right)-1\right)}^{2} = 2 \left(det\left({∇}_{x}ψ\right)-1\right) tr \left(adj\left({∇}_{x}ψ\right){∇}_{x}{U}_{α}^{T}{J}^{mn}\right) .$

The derivative of eq. (8) with respect to the *mn-*th element of *A* is ${\partial }_{{\left[A\right]}_{mn}} {\left(det\left({∇}_{x}ψ\right)-1\right)}^{2} = 2 \left(det\left({∇}_{x}ψ\right)-1\right) tr \left(adj\left({∇}_{x}ψ\right){J}^{mn}\right) .$

### Affinity

The affinity or bending energy in eq. (9) is used to impose smoothness of deformation. It penalizes any local deformation that is not affine, hinting the name. As shown in eq. (9), the penalty only depends on *W_{α}* and the derivative with respect to *W_{α}* is ${\partial }_{{W}_{α}} {R}_{A} = {K}_{α} {W}_{α} + {K}_{α}^{T} {W}_{α} .$

### Topology preservation

The inverse consistency constraint in eq. (10) is used to impose topology preservation. Since the derivation of the derivatives of ∥*ψ'* ∘ *ψ*(*x*) - *x*∥₂ and the derivatives of ∥*ψ* ∘ *ψ'*(*y*) - *y*∥₂ are similar, only the derivatives of the former with respect to the parameters of *ψ* are provided. The derivatives of the former with respect to the parameters of *ψ'* can be computed in the same manner. The derivative of ∥*ψ'* o *ψ*(*x*) - *x*∥₂ with respect to the *mn-*th element of *W_{α}* is, $\begin{matrix}{\partial }_{{\left[{W}_{α}\right]}_{mn}} {\left‖ψ′∘ψ\left(x\right)-x\right‖}_{2} \\ = {{\sum }_{i = 1}^{d} 2 {\left[ψ′∘ψ\left(x\right)-x\right]}_{i} \left[{U}_{α}{J}^{mn}A′+{U}_{α}{J}^{mn}{\left[{∇}_{ψ \left(x\right)}{U}_{α}^{′}\left(ψ\left(x\right)\right)\right]}^{T}W{′}_{α}\right]}_{i}\end{matrix}$ where *A'* and *W'_{α}* is the affine and warp coefficients of *ψ'*, and ${U}_{α}^{′} \left(ψ\left(x\right)\right)$ is the basis function of *ψ*(*x*) and the control points of *ψ'*.

The derivative of ∥*ψ'* o *ψ*(*x*) - *x*∥₂ with respect to the *mn-*th element of *A* is, $\begin{matrix}{\partial }_{{\left[A\right]}_{mn}} {\left‖ψ′∘ψ\left(x\right)-x\right‖}_{2} \\ = {\sum }_{i = 1}^{d} 2 {\left[ψ′∘ψ\left(x\right)-x\right]}_{i} {\left[{xJ}^{mn}A′+{\sum }_{α} {xJ}^{mn} {\left({∇}_{ψ \left(x\right)}{U}_{α}^{′}\left(ψ\left(x\right)\right)\right)}^{T} {W}_{α}^{′}\right]}_{i} .\end{matrix}$

The derivative with respect to *m*-th element of *t* is, ${\partial }_{{\left[t\right]}_{m}} {\left‖ψ′∘ψ\left(x\right)-x\right‖}_{2} = {\sum }_{i = 1}^{d} 2 {\left[ψ′∘ψ\left(x\right)-x\right]}_{i} {\left[{J}^{m}A′+{\sum }_{α} {J}^{m} {\left[{∇}_{ψ \left(x\right)}{U}_{α}^{′}\left(ψ\left(x\right)\right)\right]}^{T} W {′}_{α}\right]}_{i} .$

### Active control point scheme

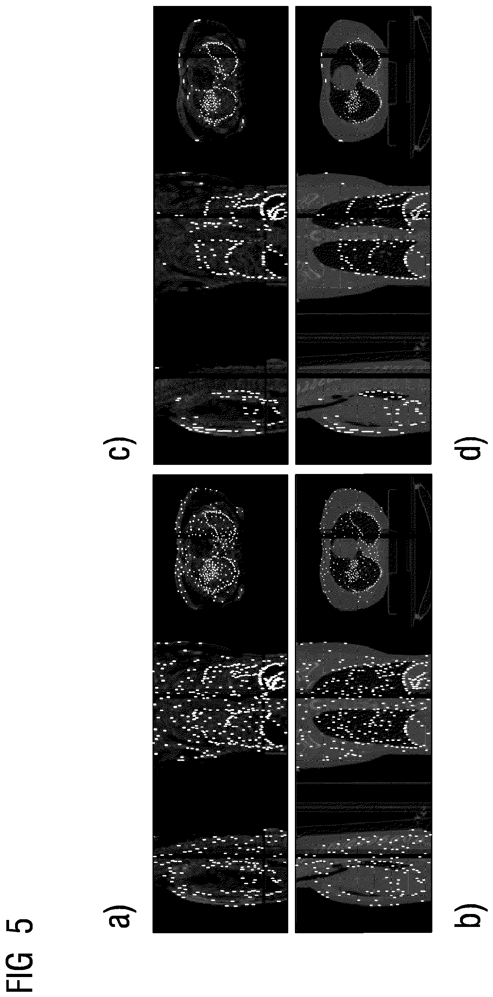

The location of control points is important for the accuracy of the deformable registration since displacements (e.g., *W_{α}*) are only defined at control point locations. This means that to accurately resolve deformation, more control points should be distributed around areas exhibiting misalignment, and no control point should (or needs to) be defined in areas that are well aligned. It is common knowledge that at the start of registration, most areas in images are misaligned, and as registration progresses misalignment in some areas will be improved or mitigated. With this knowledge, the inventive technique uses a scheme to select active control points per optimization iteration and/or per multiresolution level based on point-wise image dissimilarity metrics. Besides MSE, examples of point-wise dissimilarity metrics includes the L² distance between modality independent neighborhood descriptors (MINDs) [8], as exemplarily depicted in Figs. 5(a) and (c), mean absolute error, and/or negative of GI.

Fig. 5 shows an example of adaptive control point distributions. The first (and/or coarsest) multiresolution level can involve a relatively dense distribution of control points (marked in particular as light dots in Fig. 5) based on thresholding of image dissimilarity metrics, such as the L² distance between modality independent neighborhood descriptors [8]. Fig. 5(a) shows active control points (as light dots) overlaid on an L² distance between modality independent neighborhood descriptors (MIND) at the coarsest level of spatial resolution. Fig. 5 (b) shows active control points (as light dots) overlaid on the fixed image (e.g., the first medical image) at the coarsest level of spatial resolution. Fig. 5(c) shows a reduced number of active control points overlaid on the L² distance between modality independent neighborhood descriptors (MIND) at the next finer level of spatial resolution. Fig. 5(d) shows a reduced number of active control points overlaid on the fixed image (e.g., the first medical image) at the next finer level of spatial resolution.

Figs. 5(a) and 5(b) show the active control points at the same coarsest level of spatial resolution overlaid on the MIND map and on the fixed image (and/or the first medical image), respectively. Thus, they show the same dense set of control points.

Figs. 5(c) and 5(d) show the active control points at the next finer level of spatial resolution on the MIND map and fixed image (and/or the first medical image), respectively. Thus, Fig. 5(c) shows a sparser set of active control points compared to Fig. 5(a) on the MIND map, and the similarly for Fig. 5(d) and 5(b) for the overlay on the fixed image.

Except for minor image artefacts in the production of Figs. 5(a) to 5(d) for this disclosure, the control points the same level look exactly the same on the MIND map and on the fixed image. The same control points at the coarsest level are overlaid on Figs. 5 (a) and 5(b), which are different from the same control points at the next finer level overlaid on Figs. 5 (c) and 5(d).

After loading the first medical image and the second medical image (also: the image pair to be registered; e.g., according to the steps S102 and S104) and before running the registration optimization (e.g., according to the step S106-O), the algorithm computes an image dissimilarity map (e.g., according to the step S106-D), like the ones exemplarily depicted in Figs. 5(a) and 5(c). Based on simple thresholding followed by farthest point sampling or any other more sophisticated point sampling approaches, (e.g., according to the step S106-S) the algorithm selects control points from locations exhibiting high image dissimilarity, as exemplarily marked by the light dots in Fig. 5. Fig. 5 shows light control points overlaid on maps of MIND dissimilarity (in Figs. 5(a) and 5(c)) and on a fixed image (such as the first medical image; in Figs. 5(b) and 5(d)). Initially, the (e.g., first and second) images are mostly misaligned, resulting in a large number of control points as shown in Figs. 5(a) and 5(b). At later optimization iterations and/or multiresolution levels, active control points can be further sampled from previously densely defined control points based on the same image dissimilarity metric. As shown in Figs. 5(c) and 5(d), the number of (in particular active) control points is reduced relative to the ones shown in Figs. 5(a) and 5(b).

By allowing a set of active control points to be a subset of previously densely defined control points, all derivative calculations defined in previous sections involve a smaller memory footprint. Let 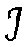 = {*..., k,* ... } be a set of indices indexing active control points in $C = {\left\{{c}_{i}\right\}}_{i = 1}^{N}$. In other words, 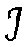 ⊆ {1,2,3, ... *N*}*.* The following terms in the above derivative calculcations can be modified to smaller matrices:
- From $U \left(x, {s}_{α}\right) ∈ {ℝ}^{1 \times N}$ to $U \left(x, {s}_{α}\right) \left[J\right] ∈ {ℝ}^{1 \times \left|J\right|}$ if *U*(*x, s_{α}*) has been precomputed; otherwise, Algorithm II and III can be evaluated for a smaller set of control points;
- From ${W}_{α} ∈ {ℝ}^{N \times d}$ to ${W}_{α} \left[J\right] ∈ {ℝ}^{\left|J\right| \times d}$, i.e., indexing into displacements associated with the active control points;
- From ${∇}_{x} U ∈ {ℝ}^{N \times d}$ to ${∇}_{x} U \left[J\right] ∈ {ℝ}^{\left|J\right| \times d}$, i.e., indexing into displacements associated with the active control points.

Adaptive selection of active control points can be enabled per optimization iteration and/or per multiresolution level. The former may involve expensive and frequent calculation of image dissimilar maps. As such, the latter may be a more appealing option.

Conventional non-learning iterative approaches are slow and unable to resolve large motion (and/or large deformation), model abrupt change in motion, and model tissue-dependent motion. Although DL approaches only involve a single forward pass, its ability to resolve deformation, so far, is inferior to non-learning approaches. Neural-field methods demonstrated improved performance over DL, but like DL, performance is limited by the memory of computing units (e.g., CPU and/or GPU memory). Since DL and neural-field methods cannot operate on native image resolution (or less coarse resolution), they struggle to resolve motion (and/or deformation) of detailed structures compared to non-learning approaches.

As non-learning approaches are still superior in terms of registration accuracy and memory usage, the inventive technique makes use of non-learning and parallelizes its calculation. Unlike prior work on parallelization of non-learning deformable registration, the inventive deformation model is parametric with the ability to capture varying degrees of deformation and to model abrupt change in motion magnitude and direction (see, e.g., eq. (1) of multiple compactly-supported RBF). The inventive technique advantageously incorporates an adaptive control point distribution scheme to improve computation efficiency and registration accuracy. The inventive technique may be additionally equipped with priors to model sliding motion between organ interface, to model tissue dependent motion (e.g., rigid motion of bony structures, and/or motion of incompressible tissue), and to preserve topology (e.g., by local diffeomorphism). Analytical gradients of these data terms and penalties are, according to the inventive technique, used to further speed up optimization while reducing memory consumption.

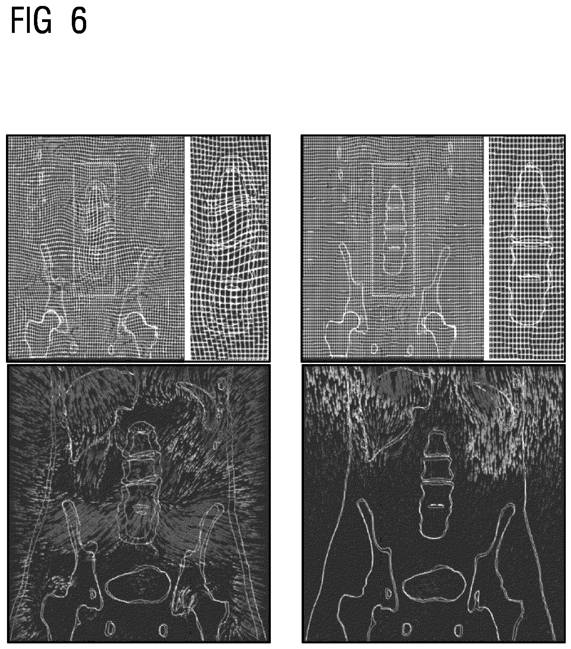

Input to the algorithms, algorithm parameters, and speed of computation may be characteristic for the inventive technique. Its output displacement field could further help identify whether the algorithm has been used since the algorithm estimates tissue-dependent deformation, with clear visualization of sliding motion, rigid motion of bone, nearly independent motion of organ in body cavities as exemplarily depicted in Fig 6.

Fig. 6 exemplarily shows tissue dependent deformation. On the top left, a conventional unconstrained approach failed to maintain rigidity of vertebrae, whereas the inventive technique on the top right preserved the rigidity of bone while allowing deformation of surrounding soft tissue. On the bottom left, the conventional unconstrained approach failed to model motion of liver and spleen due to respiratory motion in dual energy CT imaging, whereas the inventive technique on the bottom right can capture sliding motion of liver and spleen well.

Independently of the grammatical term usage, individuals with male, female or other gender identities are included within the term.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantages which are described with respect to a particular embodiment of present invention or with respect to a particular figure are, wherever applicable, also advantages of other embodiments of the present invention.

Cited prior art, which is incorporated herein by reference
[1] X. Li, et al. Neural Graphics Primitives-based Deformable Image Registration for On-the-fly Motion Extraction, 2024
[2] X. Gu, et al. Implementation and evaluation of various demons deformable image registration algorithms on a GPU, Phys. Med. Biol., 2009
[3] K. PunithaA GPU-Accelerated Deformable Image Registration Algorithm With Applications to Right Ventricular Segmentation, IEEE Access, 2017
[4] S. Ekstrom, et al. Faster dense deformable image registration by utilizing both CPU and GPU, Journal of Medical Imaging, 8(1): 2021
[5] P. Muyan-Ozcelik, et al. Fast Deformable Registration on the GPU: A CUDA Implementation of Demons, IEEE ICCSA, 2008
[6] H. Mousazadeh, et al. GPU implementation of a deformable 3D image registration algorithm, 2011 # finite element method
[7] J. P. Pluim, et al. Image Registration by Maximization of Combined Mutual Information and Gradient Information, MICCAI 2000, pp 452-461
[8] M. P. Heinrich, et al. MIND: Modality independent neighbourhood descriptor for multimodal deformable registration, Med. Imag. Anal., 16(7):1423-35,2012

## Claims

1. Computer-implemented method (100) for providing a deformable registration result of a second medical image to a first medical image, comprising the method steps of:
- Receiving (S102) a first medical image, the first medical image comprising at least one anatomical structure;
- Receiving (S104) a second medical image, wherein the second medical image comprises the at least one anatomical structure;
- Performing (S106) nested iterations, wherein the nested iterations comprise an iteration over levels of spatial resolution with increasing spatial resolution, and wherein the nested iterations further comprise, at each level of spatial resolution, an iteration regarding a set of deformation parameters of an analytic deformable registration function for registering a deformation of the received (S104) second medical image to the received (S102) first medical image, the nested iterations comprising for a predetermined level of spatial resolution:
o Determining (S106-D) an image dissimilarity between the received (S102) first medical image and the received (S104) second medical image, wherein the image dissimilarity is determined per unit volume, or per unit area, related to the predetermined level of spatial resolution;
o Selecting (S106-S) a plurality of active control points, wherein the active control points are located in regions of the first medical image and/or of the second medical image, in which the determined (S106-D) image dissimilarity exceeds, or at least equals to, a, in particular level-specific, predefined image dissimilarity threshold value;
o Iteratively optimizing (S106-O) the set of deformation parameters by iteratively optimizing an objective function, wherein the objective function is a differentiable function of the analytic deformable registration function, wherein the analytic deformable registration function comprises a sum over analytic functions centered at the selected (S106-S) active control points; and
- The iteration over levels of spatial resolution comprising:
o Proceeding (S106-P) to a further predetermined level of spatial resolution, which has a finer level of spatial resolution and/or a smaller unit volume, or unit area, than the previous predetermined level of spatial resolution, and repeating the steps of determining (S106-D) an image dissimilarity, selecting (S106-S) a plurality of active control points, and iteratively optimizing (S106-O) the set of deformation parameters at the further predetermined level of spatial resolution; and
o Terminating (S106-T) the nested iterations if, for a predetermined level of spatial resolution with finest spatial resolution and/or with a predetermined smallest size of unit volume, or unit area, the set of deformation parameters is iteratively optimized (S106-O); and
- Providing (S108) a deformable registration result of registering the second medical image to the first medical image, wherein the deformable registration result comprises utilizing the, after the terminating (S106-T) optimized, set of deformation parameters in the deformable registration function.

2. Method (100) according to claim 1, wherein the first medical image and the second medical image are acquired by means of independently selected medical imaging modalities.

3. Method (100) according to any of the preceding claims, wherein the first medical image and the second medical image are acquired by means of the same medical imaging modality.

4. Method (100) according to any of the preceding claims, wherein the step of iteratively optimizing (S106-O) the set of deformation parameters is performed using parallel computing, wherein the parallel computing comprises distributing the iteratively optimizing (S106-O) according to the selected (S106-S) active control points across parallel computing resources.

5. Method (100) according to any of the preceding claims, wherein the plurality of active control points is adaptively selected (S106-S):
- Per iterative optimization step or after a predetermined number of iterative optimization steps, in particular at a predetermined level of spatial resolution; and/or
- Per predetermined level of spatial resolution.

6. Method (100) according to any of the preceding claims, wherein selecting (S106-S) the plurality of active control points is based on point sampling, optionally
wherein performing the iteration over, in particular finer, levels of spatial resolution comprises repeating point sampling.

7. Method (100) according to any of the preceding claims, wherein the deformable registration function comprises:
- A spline function, in particular a B-spline function; and/or
- A sum over basis functions, in particular radial basis functions, RBFs, and more particularly compactly-supported RBFs, CSRBFs.

8. Method (100) according to any of the preceding claims, wherein the set of deformation parameters comprises an affine transformation matrix, an offset, a set of warp coefficients, and/or a set of widths of support of basis functions.

9. Method (100) according to any of the preceding claims, wherein the set of deformation parameters and/or the selected plurality of, in particular active, control points are stored in a shared memory.

10. Method (100) according to any of the preceding claims, wherein the objective function comprises:
- a measure of a point cloud similarity; and/or
- a measure of an image similarity.

11. Method (100) according to any of the preceding claims, wherein optimizing the objective function comprises imposing a set of physical constraints comprises at least one physical constraint, optionally
wherein imposing the set of physical constraints comprises including corresponding physical constraint terms in the objective function; and/or
wherein imposing the set of physical constraints comprises using corresponding Lagrange multipliers for the optimization.

12. Method (100) according to the directly preceding claim, wherein the set of physical constraints comprising the at least one physical constraint comprises:
an affinity or bending energy constraint;
a landmark matching;
a sliding motion constraint;
a bone rigidity constraint;
a tissue incompressibility constraint; and/or
a topology preservation constraint.

13. Method (100) according to any of the preceding claims, wherein a dissimilarity of an image or a point cloud is determined by:
- a mean distance, MD;
- a mean squared error, MSE;
- gradient information; and/or
- combined mutual information.

14. Method (100) according to any of the preceding claims, wherein receiving (S102) the first medical image and receiving (S104) the second medical image comprises receiving a segmentation mask, in particular a multi-mask, in relation to the first medical image and in relation to the second medical image, respectively.

15. Method (100) according to any of the preceding claims, wherein performing (S106) the nested iterations further comprises the steps of:
- receiving (S106-I) an indication of a downsampling factor and/or of an initial predetermined level of spatial resolution;
- downsampling (S106-DS) the first medical image and/or the second medical image to arrive at the initial predetermined level of spatial resolution;
- performing an upsampling (S106-US) of the first medical image and/or the second medical image to arrive at the further predetermined level of spatial resolution.

16. Use of the method (100) according to any of the preceding claims, for support of an image-guided intervention, for treatment monitoring, for image comparisons, and/or for radiotherapy planning.

17. Computing device (200) for providing a deformable registration result of a second medical image to a first medical image, comprising:
- A first input interface (202) configured for receiving a first medical image, the first medical image comprising at least one anatomical structure;
- A second input interface (204) configured for receiving a second medical image, wherein the second medical image comprises the at least one anatomical structure;
- A nested iterations module (206) configured for performing nested iterations, wherein the nested iterations comprise an iteration over levels of spatial resolution with increasing spatial resolution, and wherein the nested iterations further comprise, at each level of spatial resolution, an iteration regarding a set of deformation parameters of an analytic deformable registration function for registering a deformation of the received second medical image to the received first medical image, the nested iterations comprising for a predetermined level of spatial resolution:
∘ Determining an image dissimilarity between the received first medical image and the received second medical image, wherein the image dissimilarity is determined per unit volume, or per unit area, related to the predetermined level of spatial resolution;
∘ Selecting a plurality of active control points, wherein the active control points are located in regions of the first medical image and/or of the second medical image, in which the determined image dissimilarity exceeds, or at least equals to, a, in particular level-specific, predefined image dissimilarity threshold value;
∘ Iteratively optimizing the set of deformation parameters by iteratively optimizing an objective function, wherein the objective function is a differentiable function of the analytic deformable registration function, wherein the analytic deformable registration function comprises a sum over analytic functions centered at the selected (S106-S) active control points; and
- The iteration over levels of spatial resolution comprising:
∘ Proceeding to a further predetermined level of spatial resolution, which has a finer spatial resolution of spatial resolution and/or a smaller unit volume, or unit area, than the previous predetermined level of spatial resolution, and repeating the steps of determining an image dissimilarity, selecting a plurality of active control points, and iteratively optimizing the set of deformation parameters at the further predetermined level of spatial resolution; and
∘ Terminating the nested iterations if, for a predetermined level of spatial resolution with finest spatial resolution and/or with a predetermined smallest size of unit volume, or unit area, the set of deformation parameters is iteratively optimized; and
- An output interface (208) configured for providing a deformable registration result of registering the second medical image to the first medical image, wherein the deformable registration result comprises utilizing the, after the terminating optimized, set of deformation parameters in the deformable registration function.

## Amended claims

### Amended claims in accordance with Rule 137(2) EPC.

1. Computer-implemented method (100) for providing a deformable registration result of a second medical image to a first medical image, comprising the method steps of:
- Receiving (S102) a first medical image, the first medical image comprising at least one anatomical structure;
- Receiving (S104) a second medical image, wherein the second medical image comprises the at least one anatomical structure;
- Performing (S106) nested iterations, wherein the nested iterations comprise an iteration over levels of spatial resolution with increasing spatial resolution, and wherein the nested iterations further comprise, at each level of spatial resolution, an iteration regarding a set of deformation parameters of an analytic deformable registration function for registering a deformation of the received (S104) second medical image to the received (S102) first medical image, the nested iterations comprising for a predetermined level of spatial resolution:
∘ Determining (S106-D) an image dissimilarity between the received (S102) first medical image and the received (S104) second medical image, wherein the image dissimilarity is determined per unit volume, or per unit area, related to the predetermined level of spatial resolution;
∘ Selecting (S106-S) a plurality of active control points, wherein the active control points are located in regions of the first medical image and/or of the second medical image, in which the determined (S106-D) image dissimilarity exceeds, or at least equals to, a, in particular level-specific, predefined image dissimilarity threshold value, wherein selecting (S106-S) the plurality of active control points is based on point sampling, whereby active control points are sampled from a number of control-point candidates, for which the determined (S106-D) image dissimilarity exceeds, or at least equals to the predefined image dissimilarity threshold value;
∘ Iteratively optimizing (S106-O) the set of deformation parameters by iteratively optimizing an objective function, wherein the objective function is a differentiable function of the analytic deformable registration function, wherein the analytic deformable registration function comprises a sum over analytic functions centered at the selected (S106-S) active control points, wherein the step of iteratively optimizing (S106-O) the set of deformation parameters is performed using parallel computing, wherein the parallel computing comprises distributing the iteratively optimizing (S106-O) according to the selected (S106-S) active control points across parallel computing resources, wherein the set of deformation parameters and the selected (S106-S) plurality of active control points are stored in a shared memory;
- The iteration over levels of spatial resolution comprising:
∘ Proceeding (S106-P) to a further predetermined level of spatial resolution, which has a finer level of spatial resolution and/or a smaller unit volume, or unit area, than the previous predetermined level of spatial resolution, and repeating the steps of determining (S106-D) an image dissimilarity, selecting (S106-S) a plurality of active control points, and iteratively optimizing (S106-O) the set of deformation parameters at the further predetermined level of spatial resolution, wherein performing the iteration over, in particular finer, levels of spatial resolution comprises repeating point sampling for selecting (S106-S) the plurality of active control points, and wherein the selected (S106-S) plurality of active control points is iteratively reduced; and
∘ Terminating (S106-T) the nested iterations if, for a predetermined level of spatial resolution with finest spatial resolution and/or with a predetermined smallest size of unit volume, or unit area, the set of deformation parameters is iteratively optimized (S106-O); and
- Providing (S108) a deformable registration result of registering the second medical image to the first medical image, wherein the deformable registration result comprises utilizing the, after the terminating (S106-T) optimized, set of deformation parameters in the deformable registration function.

2. Method (100) according to claim 1, wherein the first medical image and the second medical image are acquired by means of independently selected medical imaging modalities.

3. Method (100) according to any of the preceding claims, wherein the first medical image and the second medical image are acquired by means of the same medical imaging modality.

4. Method (100) according to any of the preceding claims, wherein the plurality of active control points is adaptively selected (S106-S):
- Per iterative optimization step or after a predetermined number of iterative optimization steps, in particular at a predetermined level of spatial resolution; and/or
- Per predetermined level of spatial resolution.

5. Method (100) according to any of the preceding claims, wherein the deformable registration function comprises:
- A spline function, in particular a B-spline function; and/or
- A sum over basis functions, in particular radial basis functions, RBFs, and more particularly compactly-supported RBFs, CSRBFs.

6. Method (100) according to any of the preceding claims, wherein the set of deformation parameters comprises an affine transformation matrix, an offset, a set of warp coefficients, and/or a set of widths of support of basis functions.

7. Method (100) according to any of the preceding claims, wherein the objective function comprises:
- a measure of a point cloud similarity; and/or
- a measure of an image similarity.

8. Method (100) according to any of the preceding claims, wherein optimizing the objective function comprises imposing a set of physical constraints, which comprises at least one physical constraint, optionally
wherein imposing the set of physical constraints comprises including corresponding physical constraint terms in the objective function; and/or
wherein imposing the set of physical constraints comprises using corresponding Lagrange multipliers for the optimization.

9. Method (100) according to the directly preceding claim, wherein the set of physical constraints comprising the at least one physical constraint comprises:
an affinity or bending energy constraint;
a landmark matching;
a sliding motion constraint;
a bone rigidity constraint;
a tissue incompressibility constraint; and/or
a topology preservation constraint.

10. Method (100) according to any of the preceding claims, wherein a dissimilarity of an image or a point cloud is determined by:
- a mean distance, MD;
- a mean squared error, MSE;
- gradient information; and/or
- combined mutual information.

11. Method (100) according to any of the preceding claims, wherein receiving (S102)the first medical image and receiving (S104) the second medical image comprises receiving a segmentation mask, in particular a multi-mask, in relation to the first medical image and in relation to the second medical image, respectively.

12. Method (100) according to any of the preceding claims, wherein performing (S106) the nested iterations further comprises the steps of:
- receiving (S106-I) an indication of a downsampling factor and/or of an initial predetermined level of spatial resolution;
- downsampling (S106-DS) the first medical image and/or the second medical image to arrive at the initial predetermined level of spatial resolution;
- performing an upsampling (S106-US) of the first medical image and/or the second medical image to arrive at the further predetermined level of spatial resolution.

13. Use of the method (100) according to any of the preceding claims, for support of an image-guided intervention, for treatment monitoring, for image comparisons, and/or for radiotherapy planning.

14. Computing device (200) for providing a deformable registration result of a second medical image to a first medical image, comprising:
- A first input interface (202) configured for receiving a first medical image, the first medical image comprising at least one anatomical structure;
- A second input interface (204) configured for receiving a second medical image, wherein the second medical image comprises the at least one anatomical structure;
- A nested iterations module (206) configured for performing nested iterations, wherein the nested iterations comprise an iteration over levels of spatial resolution with increasing spatial resolution, and wherein the nested iterations further comprise, at each level of spatial resolution, an iteration regarding a set of deformation parameters of an analytic deformable registration function for registering a deformation of the received second medical image to the received first medical image, the nested iterations comprising for a predetermined level of spatial resolution:
o Determining an image dissimilarity between the received first medical image and the received second medical image, wherein the image dissimilarity is determined per unit volume, or per unit area, related to the predetermined level of spatial resolution;
∘ Selecting a plurality of active control points, wherein the active control points are located in regions of the first medical image and/or of the second medical image, in which the determined image dissimilarity exceeds, or at least equals to, a, in particular level-specific, predefined image dissimilarity threshold value, wherein selecting the plurality of active control points is based on point sampling, whereby active control points are sampled from a number of control-point candidates, for which the determined image dissimilarity exceeds, or at least equals to the predefined image dissimilarity threshold value;
∘ Iteratively optimizing the set of deformation parameters by iteratively optimizing an objective function, wherein the objective function is a differentiable function of the analytic deformable registration function, wherein the analytic deformable registration function comprises a sum over analytic functions centered at the selected active control points, wherein the step of iteratively optimizing the set of deformation parameters is performed using parallel computing, wherein the parallel computing comprises distributing the iteratively optimizing according to the selected active control points across parallel computing resources, wherein the set of deformation parameters and the selected plurality of active control points are stored in a shared memory; and
- The iteration over levels of spatial resolution comprising:
o Proceeding to a further predetermined level of spatial resolution, which has a finer spatial resolution of spatial resolution and/or a smaller unit volume, or unit area, than the previous predetermined level of spatial resolution, and repeating the steps of determining an image dissimilarity, selecting a plurality of active control points, and iteratively optimizing the set of deformation parameters at the further predetermined level of spatial resolution, wherein performing the iteration over, in particular finer, levels of spatial resolution comprises repeating point sampling for selecting the plurality of active control points, and wherein the selected plurality of active control points is iteratively reduced; and
o Terminating the nested iterations if, for a predetermined level of spatial resolution with finest spatial resolution and/or with a predetermined smallest size of unit volume, or unit area, the set of deformation parameters is iteratively optimized; and
- An output interface (208) configured for providing a deformable registration result of registering the second medical image to the first medical image, wherein the deformable registration result comprises utilizing the, after the terminating optimized, set of deformation parameters in the deformable registration function.

15. Computing device (200) according to the directly preceding claim, further configured to perform any one of the steps, and/or comprise any one of the features, according to any one of the method claims 2 to 12.
